(19)

**Europäisches Patentamt**

**European Patent Office**

**Office européen des brevets**

(11) **EP 4 603 111 A1**

(12) **EUROPEAN PATENT APPLICATION**

(43) Date of publication:
20.08.2025 Bulletin 2025/34

(21) Application number: 24158239.4

(22) Date of filing: 16.02.2024

(51) International Patent Classification (IPC):
*A61L 27/20* (2006.01)   *A61L 27/38* (2006.01)
*A61L 27/50* (2006.01)

(52) Cooperative Patent Classification (CPC):
(C-Sets available)
**A61L 27/20; A61L 27/3808; A61L 27/3834;
A61L 27/507**                              (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Christian-Albrechts-Universität zu Kiel
24118 Kiel (DE)**

(72) Inventor: **The designation of the inventor has not
yet been filed**

(74) Representative: **Eisenführ Speiser
Patentanwälte Rechtsanwälte PartGmbB
Johannes-Brahms-Platz 1
20355 Hamburg (DE)**

Remarks:
•The complete document including Reference
Table(s) and the Sequence Listing(s) can be
downloaded from the EPO website
•Claims 16 - 22 are deemed to be abandoned due to
non-payment of the claims fees (Rule 45(3) EPC).

(54) **NOVEL BIOARTIFICIAL VASCULAR GRAFTS AND METHODS FOR THEIR FABRICATION**

(57)   The present invention relates to a novel bio-ink for preparing bioartificial vascular grafts which are highly useful for vascular and cardiovascular research and therapy, and in particular for surgical vascular and cardiovascular repair and bypass surgery. The bio-ink composition of the invention comprises a peptide-grafted alginate and smooth muscle cells, endothelial cells or cells which are capable of developing into endothelial cells. According to the invention, the peptides grafted onto the alginate are derived from an extracellular matrix protein or mimic such proteins. The invention also relates to a method for manufacturing a bioartificial vascular graft comprising the printing of the bio-ink composition of the invention. Bioartificial vascular grafts, which are obtainable by the method of the invention, are also encompassed by the invention. In yet another aspect, the invention relates to the use of the bio-ink for in vitro drug screening and as an in-vitro training model for endovascular or surgical training.

EP 4 603 111 A1

**(Cont. next page)**

(52)  Cooperative Patent Classification (CPC): (Cont.)

C-Sets
**A61L 27/20, C08L 5/04**

## Description

The present invention relates to a novel bio-ink for preparing bioartificial vascular grafts which are highly useful for vascular and cardiovascular research and therapy, and in particular for surgical vascular and cardiovascular repair and bypass surgery. The bio-ink composition of the invention comprises a peptide-grafted alginate and smooth muscle cells, endothelial cells or cells which are capable of developing into endothelial cells. According to the invention, the peptides grafted onto the alginate are derived from an extracellular matrix protein or mimic such proteins. The invention also relates to a method for manufacturing a bioartificial vascular graft comprising the printing of the bio-ink composition of the invention. Bioartificial vascular grafts, which are obtainable by the method of the invention, are also encompassed by the invention. In yet another aspect, the invention relates to the use of the bio-ink for in vitro drug screening.

## TECHNICAL BACKGROUND

Coronary or peripheral bypass surgery due to arteriosclerotic stenosis or occlusion remains one of the major medical procedures of today (Kullo et al., 2016; Head et al., 2017). Additionally, an increasing number of civilian and military vessel traumas have been reported during the previous five decades associated with an increase of vascular repair (Caps, 1998; Haney et al., 2020). In the majority of cases, autologous vein or less often arterial vessels are implanted as bypass grafts or vascular conduits.

Due to the nature of autologous approaches, the number of grafts is limited which could be decisive to treatment in case of re-operation or co-morbidities of the vascular system (Caliskan et al., 2020; Gallo et al., 2022; Xenogiannis et al., 2021). Artificial polymer grafts are still associated with poor patency rates and high infection risk or are not suitable in various treatments especially for small vessel grafting e.g., coronary bypass surgery (Twine et al., 2010). Accordingly, the ideal and rapidly available vascular graft remains a challenge of modern medicine - not only for the surgical team but also for biomedical research and health care systems.

Recent developments in tissue engineering in general and especially in 3D bioprinting have raised the legitimate hope to fabricate a bioartificial vascular graft for cardiovascular surgery (Sasmal et al., 2018; Matai et al. 2020). A number of preliminary studies have described bioprinting strategies for vessel-like structures ranging from customized to commercial, from co-axial to laser-based bioprinting and presenting a wide range of different bio-inks (Matai et al., 2020). For example, Zhang and co-workers described a bioprinted perfusable vasculature conduit fabricated from human umbilical vein smooth muscle cells (SMCs) encapsulated in sodium alginate (Zhang et al., 2015). Hong and colleagues demonstrated a vascular construct using a gelatin-tyramine bio-ink (Hong et al., 2019). A similar approach was described by Cui and co-workers demonstrating a small-diameter vasculature with smooth muscle and endothelium (Cui et al., 2019).

However, the majority of these approaches did not provide the necessary bioartificial integrity, a pipeline for clinical translation and were mainly focused on in-vitro disease modelling (Sasmal et al., 2018). Bioartificial vascular grafts for surgical implantation are mostly bioengineered from components of the extracellular matrix, are acellular and therefore lack the functional biology of human vessels (Dahl et al., 2011). In addition, limited cell sources have been described as a major problem of these tissue-engineering approaches (Ott et al., 2011). Consequently, none of these former studies has yet provided a bioartificial vascular graft combining the required biomimetic characteristics of human vessels and a strategy for clinical translation.

As a consequence, there is an unfulfilled demand in human-scale bioartificial vascular grafts which can be used in cardiovascular therapy and exert biomechanics and biomimetics that resemble human vessels, a high capability of biointegration, and low thrombogenicity. The bioartificial vascular grafts should be available by reproducible, scalable and reliable GMP processes. Moreover, the manufacturing process should be short and scalable to allow a more "bedside" orientated approach.

The present invention has overcome the problems experienced in the past and provides additional advantages as well. The present invention for the first time demonstrates that bioartificial vascular grafts having human-scale structure and functional integrity can be obtained by 3D bioprinting. The present invention provides a novel bio-ink which comprises an alginate, such as sodium alginate (SA) and one or more peptides, such as peptides derived from proteins of the extracellular matrix (EM). Endothelial or SMCs are integrated into the vessel wall as part of the printing process to create a vein-like vascular graft ready for clinical application. The grafts of the present invention exert biomechanical properties and characteristics that render them suitable for being used in clinical settings for repairing or replacing dysfunctional or diseased vessels.

## SUMMARY OF THE INVENTION

Accordingly, in a first aspect the present invention relates to a bio-ink composition which can be used for bioprinting a vascular graft, said bio-ink composition comprising:

(a) a peptide-grafted alginate; and

(b) SMCs, endothelial cells or cells which are capable of developing into endothelial cells.

The bio-ink composition according to the first aspect of the invention is suitable for being printed to a 3D bioartificial vascular graft by use of a 3D printer. Therefore, in a second aspect, the present invention relates to a method for manufacturing a bioartificial vascular graft, comprising

(a) loading the bio-ink composition according to the first aspect of the invention into a 3D printer which is capable of printing viscous hydrogels;

(b) contacting the bio-ink composition with a crosslinking solution comprising a divalent and/or trivalent cation in the 3D printer to induce crosslinking of the alginate;

(c) printing the bio-ink to obtain the bioartificial vascular graft.

In a third aspect, the present invention relates to a bioartificial vascular graft obtainable by the method according to the second aspect of the present invention. The bioartificial vascular graft according to the third aspect of the invention can be used as a vascular conduit or bypass material.

In a fourth aspect, the present invention relates to the bioartificial vascular graft according to the third aspect of the invention for use in medicine, and in particular for use in vascular or cardiovascular therapy, such as bypass surgery.

In a fifth aspect, the present invention relates to the use of a bio-ink composition according to the first aspect of the invention for manufacturing a bioartificial vascular graft, such as vascular conduit and bypass material. The bioartificial vascular graft can be used as a vascular conduit or bypass material.

In a sixth aspect, the present invention relates to the use of a bio-ink composition according to the first aspect of the invention for in vitro drug screenings or an in-vitro training model for endovascular or surgical training.

## DETAILED DESCRIPTION

The bio-ink composition of the present invention provides a 3D matrix for printing a bioartificial vessel that can be applied for research purposes, for in vitro disease modelling or in medical therapy. The matrix integrates high tensile strength, elasticity and a favorable environment for cell engraftment which are essential requirements for mimicking the characteristics of natural vessels (Matai et al., 2020). Accordingly, the bio-ink composition of the present invention is highly suitable for manufacturing of vascular conduits and bypass material.

The bio-ink of the present invention comprises, as a first component, a peptide-grafted alginate, i.e. an alginate to which one or more peptides have been covalently coupled. As used herein, the term "alginate" refers to a salt of alginic acid. Alginic acid is a polysaccharide consisting of a linear copolymer with homopolymeric blocks of (1-4)-linked $\beta$-D-mannuronate (M) and $\alpha$-L-guluronate (G) residues, respectively, covalently linked together in different sequences or blocks. The monomers may appear in homopolymeric blocks of consecutive G-residues (G-blocks), consecutive M-residues (M-blocks) or alternating M and G-residues (MG-blocks). Alginic acid naturally occurs in brown algae. It is a hydrophilic molecule which, upon hydration or interaction with divalent cations, forms a hydrogel. Metal salts of alginic acid, such as sodium alginate and calcium alginate, are in wide use for different purposes, for example as thickening agents in foods and cosmetics, as an ingredient in various pharmaceutical products, and as a biomaterial for tissue regeneration. Alginates have also been contemplated for bioprinting (Fan et al., 2013; Hu et al., 2021). Although sodium alginate exerts some beneficial physicochemical properties, such as biocompatibility, non-toxicity and non-immunogenicity, its capability of cell engraftment is limited (Fan et al., 2013; Matai et al., 2020; Hu et al., 2021). In addition, the biomechanical properties of sodium alginate are rather limited. The present invention overcomes this problem by modifying the alginate with EM protein fragments. The M/G ratio of the alginate that is used in the methods and compositions of the invention can vary between 10:1 and 1:10, and will preferably be about 10:1, 9:1, 8:1, 7:1, 6:1, 5:1, 4:1, 3:1, 2:1, and 1:1. In a preferred embodiment, the M/G ratio of the alginate that is used in the methods and compositions of the invention is about 1:1. It is also possible according to the invention to use an alginate that completely consists of a mannuronate, such as a (1-4)-linked $\beta$-D-mannuronate.

In a preferred embodiment, the alginate which is present in the bio-ink composition of the invention is sodium alginate. In another preferred embodiment, the alginate which is present in the bio-ink composition of the invention is calcium alginate. In yet another preferred embodiment, the alginate which is present in the bio-ink composition of the invention is a mixture of sodium alginate and calcium alginate.

The present invention is based, amongst others, on the insight that the positive effects of sodium alginate and EM proteins can be combined by grafting smaller peptides, e.g. peptides obtained by hydrolysis of EM proteins or artificial peptides that comprise an amino acid sequence which occurs in EM proteins, in particular collagen, onto sodium alginate to provide a grafted matrix that can be used as a bio-ink. In the bio-ink composition of the invention, the alginate is present in the form of peptide-alginate complexes in which the alginate is covalently coupled to at least one peptide, i.e., one defined type of peptide, such as a peptide derived from the hydrolysis of collagen or manufactured by peptide synthesis.

According to the invention, the peptide which is coupled to the alginate preferably comprises an amino acid sequence which is derived from an EM protein, such as a human EM protein. As used herein, "derived from" means that the amino acid sequence in the peptide occurs in identical or highly similar form in the amino acid sequence of a native EM protein. Accordingly, the peptide may comprise or consist of an amino acid sequence which is present in a naturally occurring. EM protein. Alternatively, the peptide may comprise or consist of an amino acid sequence which resembles an amino acid sequence which is present in a naturally occurring EM protein. The EM protein preferably is a human EM protein. Also encompassed by the term "derived from an EM protein" or "EM protein-derived" are peptides which structurally mimic an EM protein, preferably a human EM protein. Such peptides include peptides that are recognized by a protease that specifically recognizes the corresponding EM protein. For example, the synthetic peptide N-[3-(2-Furyl) acryloyl]-L-leucyl-glycyl-L-prolyl-L-alanine (FALGPA) which was used in the below examples resembles a partial structure of the human collagen and is hence recognized by a collagenase enzyme. In a preferred embodiment, the EM protein-derived peptide is recognized by the collagenase of Clostridium histolyticum, an enzyme that is widely commercialized in kits for measuring collagenase activity.

The EM protein from which the one or more peptides can be derived are preferably selected from the group consisting of collagen, tropocollagen, elastin, tropoelastin, fibrillin I, fibrillin II, fibrillin III, fibrillin IV, fibronectin vitronectin and laminin. In one embodiment, the peptide used in the bio-ink composition of the invention comprises a peptide which is derived from a collagen protein. The collagen protein can be a collagen I protein, II protein, a collagen III protein, a collagen IV protein, a collagen V protein, a collagen VI protein, a collagen VII protein, a collagen VIII protein, a collagen IX protein, a collagen X protein, a collagen XI protein, a collagen XII protein, a collagen XIII protein, a collagen XIV protein, a collagen XV protein, a collagen XVI protein, a collagen XVII protein, a collagen XVIII protein, a collagen XIX protein, a collagen XX protein, a collagen XXI protein, a collagen XXII protein, a collagen XXIII protein, a collagen XXIV protein, a collagen XXV protein, a collagen XXVI protein, a collagen XXVII, a collagen XXVIII, or a collagen XIX. It is particular preferred that the peptide is derived from a collagen I or collagen II protein, and more preferably from the human collagen I or collagen II protein. In another embodiment, the peptide used in the bio-ink composition is derived from a proteoglycan, preferably, from an aggrecan, decorin, or perlecan.

In a particularly preferred embodiment, the peptide in the peptide-grafted alginate is derived from collagen, i.e., the peptide comprises or consists of an amino acid sequence that occurs in collagen, preferably human collagen, or is highly similar to an amino acid sequence that occurs in collagen.

In another embodiment, the alginate is covalently coupled to a combination of different peptides, such as 2, 3, 4, 5, 6, 7, 8, 9, 10 or more peptides, such as EM protein-derived peptides. For example, the alginate may be covalently coupled to a first EM-derived peptide and a second EM-derived peptide wherein both peptides differ from each other in terms of their amino acid sequence. Thus, the invention also relates to a bio-ink composition which comprises a peptide-grafted alginate, in which the alginate is grafted with 2 or more different peptides.

In a preferred embodiment, the alginate is present in the form of peptide-alginate complexes in which the alginate is covalently coupled to a first collagen-derived peptide and a second collagen-derived peptide which differs from the first peptide in terms of its amino acid sequence. For example, in one embodiment, the first collagen-derived peptide is derived from collagen I, and the second collagen-derived peptide is derived from collagen II. In another embodiment, the first collagen-derived peptide is derived from collagen I, and the second collagen-derived peptide is derived from collagen III. In yet another embodiment, the first collagen-derived peptide is derived from collagen I, and the second collagen-derived peptide is derived from collagen IV. In yet another embodiment, the first collagen-derived peptide is derived from collagen I, and the second collagen-derived peptide is derived from collagen V. In yet another embodiment, the first collagen-derived peptide is derived from collagen I, and the second collagen-derived peptide is derived from collagen VI. In yet another embodiment, the first collagen-derived peptide is derived from collagen I, and the second collagen-derived peptide is derived from collagen VII. In yet another embodiment, the first collagen-derived peptide is derived from collagen I, and the second collagen-derived peptide is derived from collagen VIII. In yet another embodiment, the first collagen-derived peptide is derived from collagen I, and the second collagen-derived peptide is derived from collagen IX. In yet another embodiment, the first collagen-derived peptide is derived from collagen I, and the second collagen-derived peptide is derived from collagen X.

Alternatively, the alginate may also be covalently coupled to a combination of a first collagen-derived peptide and a second peptide which is derived from another EM protein, i.e., an EM protein other than collagen. The second EM-derived peptide can be derived from any of the EM proteins referred to above. In one embodiment, the second peptide is derived from the EM protein laminin. In another embodiment, the second peptide is derived from the EM protein elastin. In still another embodiment, the second peptide is derived from the EM protein tropoelastin. In another embodiment, the second peptide is derived from the EM protein fibrillin I, fibrillin II, fibrillin III, or fibrillin IV. In still another embodiment, the second peptide is derived from the EM protein fibronectin or vitronectin.

Each of the one or more peptides which are covalently coupled to the alginate preferably has a size of 3-50 amino acids. In one embodiment, each of the peptides has a size of 3-45 amino acids, 3-40 amino acids, 3-35 amino acids, 3-30 amino acids, 3-25 amino acids, 3-20 amino acids, 3-15 amino acids, 3-10 amino acids, 3-9 amino acids, 3-8 amino acids, 3-7 amino acids, 3-6 amino acids, 3-5 amino acids, or 3-4 amino acids. In another embodiment, each of the peptides has a size of 4-45 amino acids, 4-40 amino acids, 4-35 amino acids, 4-30 amino acids, 4-25 amino acids, 4-20 amino acids, 4-15 amino acids, 4-10 amino acids, 4-9 amino acids, 4-8 amino acids, 4-7 amino acids, 4-6 amino acids, or 4-5 amino acids. Preferably, each of the peptides has a size of 3 amino acids, 4 amino acids, 5 amino acids, 6 amino acids, 7 amino acids, 8 amino acids, 9 amino acids, or 10 amino acids. A size of 4-8 or 4-10 amino acids is particularly preferred. It is particular preferred that the above sizes refer to a peptide which is derived from a collagen protein, more preferably a collagen I or collagen II protein, such as the human collagen I or collagen II protein.

In one preferred embodiment, the peptide in the bio-ink composition of the present invention is derived from a collagen I protein and comprises or consists of the tetramer sequence $NH_2$-L-G-P-A-COOH (SEQ ID NO:1). In another preferred embodiment, the peptide in the bio-ink composition is derived from a collagen I protein and comprises or consists of the sequence N-(3-[2-Furyl]acryloyl)-L-G-P-A (SEQ ID NO:2). In another preferred embodiment, the peptide in the bio-ink composition is derived from laminin and comprises or consists of the hexamer sequence $NH_2$-S-I-K-V-A-V-COOH (SEQ ID NO:3). In yet another preferred embodiment, the peptide in the bio-ink composition is derived from elastin and comprises or consists of the hexamer sequence $NH_2$-V-G-V-A-P-G-COOH (SEQ ID NO:4). In yet another preferred embodiment, the peptide in the bio-ink composition is derived from laminin and comprises or consists of the hexamer sequence $NH_2$-V-G-V-A-P-G-COOH (SEQ ID NO:5). In yet another preferred embodiment, the peptide in the bio-ink composition comprises or consists of the $NH_2$-R-G-D-COOH. In yet another preferred embodiment, the peptide in the bio-ink composition comprises or consists of the $NH_2$-K-Q-A-G-D-V-COOH (SEQ ID NO:6). Such peptides are discussed in more detail in Hashimoto et al. 2004. Without wishing to be bound by theory, it is assumed that EPCs, SMCs, and endothelial cells recognize these sequences such that their integration into the alginate matrix is promoted.

It is of course also possible to produce, either synthetically or by recombinant expression, hybrid peptides which encompass two or more amino acid sequences which are derived from different EM proteins. For example, hybrid peptides can be produced which combine peptide sequences derived from laminin and elastin. Such peptides include, e.g., $NH_2$-K-S-I-R-V-A-V-A-P-G-COOH (SEQ ID NO:7) and $NH_2$-K-S-I-R-V-G-V-G-P-G-COOH (SEQ ID NO:8). The skilled person will be readily able to synthesize or recombinantly express hybrid peptides which combine sequences from two or more EM proteins.

According to the invention, it is preferred that the alginate is present in the form of peptide-alginate complexes in which the alginate is covalently coupled to one or more peptides that comprise or consist of the amino acid sequence set forth in SEQ ID NOs:1-8 or a variant of any of these. As used herein, an amino acid sequence is considered a "variant" if it differs from one of the amino acid sequences set forth in SEQ ID NOs:1-8 by no more than 2 amino acid exchanges, and more preferably by no more than 1 amino acid exchange. Thus, it is preferred in one embodiment that the alginate is coupled to a peptide that comprises or consists of the sequence of SEQ ID NO:1 or a sequence that differs from the sequence of SEQ ID NO:1 by 1 or 2 amino acid exchanges. In another embodiment the alginate is coupled to a peptide that comprises or consists of the sequence of SEQ ID NO:2 or a sequence that differs from the sequence of SEQ ID NO:2 by 1 or 2 amino acid exchanges. In yet another embodiment the alginate is coupled to a peptide that comprises or consists of the sequence of SEQ ID NO:3 or a sequence that differs from the sequence of SEQ ID NO:3 by 1 or 2 amino acid exchanges. In another embodiment the alginate is coupled to a peptide that comprises or consists of the sequence of SEQ ID NO:4 or a sequence that differs from the sequence of SEQ ID NO:4 by 1 or 2 amino acid exchanges. In another embodiment the alginate is coupled to a peptide that comprises or consists of the sequence of SEQ ID NO:5 or a sequence that differs from the sequence of SEQ ID NO:5 by 1 or 2 amino acid exchanges. In another embodiment the alginate is coupled to a peptide that comprises or consists of the sequence of SEQ ID NO:6 or a sequence that differs from the sequence of SEQ ID NO:6 by 1 or 2 amino acid exchanges. In another embodiment the alginate is coupled to a peptide that comprises or consists of the sequence of SEQ ID NO:7 or a sequence that differs from the sequence of SEQ ID NO:7 by 1 or 2 amino acid exchanges. In another embodiment the alginate is coupled to a peptide that comprises or consists of the sequence of SEQ ID NO:8 or a sequence that differs from the sequence of SEQ ID NO:8 by 1 or 2 amino acid exchanges

The one or more EM-derived peptides can be coupled to the alginate, preferably sodium or calcium alginate, by different chemical methods for linking peptides to polysaccharides. For example, the peptide or peptides can be covalently coupled to the alginate via an amide linkage in the presence of 1-ethyl-(dimethylaminopropyl) carbodiimide and N-hydroxy sulfosuccinimide. As described in the below examples, the carbodiimide reaction was used to mediate the crosslinking between sodium alginate and an EM-derived peptide, the FALGPA peptide (N-(3-[2-Furyl]acryloyl)-L-G-P-A SIGMA-Aldrich). 0.35 g sodium alginate was dissolved in 0.2 M MES (2-(N-morpholino) ethanesulfonic acid) buffer containing 0.3 M NaCl, pH 5.5. Then, 0.035 g N-hydroxy sulfosuccinimide (NHS) and 0.035 g N-(3-Dimethylaminopropyl)-N-ethylcarbodiimide hydrochloride (EDC) were added step by step into the solution at room temperature with magnetic stirring for 20 min. The pH was slowly increased to 7 and then peptides were added into the solution facilitating the grafting for 8 h. The 1 % alginate derivative solution so produced was purified by dialysis through a 6-8 KD molecular weight cut-off dialysis tube for three days. The dialyzed product was finally freeze-dried with LyoVac GT2 to obtain a purified alginate derivative powder. The dried samples were stored at -20°C.

The overall amount of peptide which is coupled to the alginate in the peptide-alginate complexes can be between 1% and 25%, based on the overall weight of β-D-mannuronate in the alginate (w/w). More preferably, the amount of peptide in the peptide-alginate complexes is between 1% and 20% (w/w), between 1% and 15% (w/w), between 1% and 12% (w/w), or between 1% and 10% (w/w), based on the overall weight of β-D-mannuronate in the alginate. More preferably, the amount of peptide in the peptide-alginate complexes is between 2% and 25% (w/w), between 2% and 20% (w/w), between 2% and 15% (w/w), between 2% and 12% (w/w), or between 2% and 10% (w/w), based on the overall weight of β-D-mannuronate in the alginate. More preferably, the amount of peptide in the peptide-alginate complexes is between 3% and 25% (w/w), between 3% and 20% (w/w), between 3% and 15% (w/w), between 3% and 12% (w/w), or between 3% and 10% (w/w), based on the overall weight of β-D-mannuronate in the alginate. More preferably, the amount of peptide in the peptide-alginate complexes is between 4% and 25% (w/w), between 4% and 20% (w/w), between 4% and 15% (w/w), between 4% and 12% (w/w), or between 4% and 10% (w/w), based on the overall weight of β-D-mannuronate in the alginate.

Stated differently, the overall amount of peptide which is coupled to the alginate in the peptide-alginate complexes can be between 0.5% and 12.5% (w/w), based on the overall weight of the alginate. More preferably, the amount of peptide in the peptide-alginate complexes is between 0.5% and 10% (w/w), between 0.5% and 7.5% (w/w), between 0.5% and 6% (w/w), or between 0.5% and 5% (w/w), based on the overall weight of the alginate. More preferably, the amount of peptide in the peptide-alginate complexes is between 1% and 12.5% (w/w), between 1% and 10% (w/w), between 1% and 7.5% (w/w), between 1% and 6% (w/w), or between 1% and 5% (w/w), based on the overall weight of the alginate. More preferably, the amount of peptide in the peptide-alginate complexes is between 1.5% and 12.5% (w/w), between 1.5% and 10% (w/w), between 1.5% and 7.5% (w/w), between 1.5% and 6% (w/w), or between 1.5% and 5% (w/w), based on the overall weight of the alginate. More preferably, the amount of peptide in the peptide-alginate complexes is between 2% and 12.5% (w/w), between 2% and 10% (w/w), between 2% and 7.5% (w/w), between 2% and 6% (w/w), or between 2% and 5% (w/w), based on the overall weight of the alginate.

The presence and percentage of coupled peptides in the alginate can be confirmed by differences in the absorbances compared to an unmodified alginate control, as further described in the below Example 1. Alternatively, the presence and percentage of coupled peptides in the alginate can be confirmed by mass spectroscopy, as described in Fan et al. 2013.

The bio-ink composition of the invention comprises, as a second component, SMCs, endothelial cells or cells which are capable of developing into endothelial cells. The cells are preferably autologous in relation to the subject that shall receive the bioartificial vascular graft, i.e., the cells have been derived from the subject that shall receive the printed graft. However, allogenic cells may also be used, i.e., cells obtained from a subject of the same species not being the patient to be treated. For example, hypoimmunogenic allogenic endothelial cells can be used in order to avoid a strong immunogenic reaction in the patient receiving the graft.

In one embodiment, the cells that are added to the bio-ink composition of the invention are endothelial cells. As used herein, the term "endothelial cells" generally refers to cells that line the interior surface of blood vessels or lymphatic vessels. Preferably, the endothelial cells present in the bio-ink of the invention are vascular endothelial cells, i.e., cells that form the inner cellular lining of blood vessels, including arteries, veins and capillaries. Endothelial cells express the cells surface markers CD31, CD34 and ICAM1 and can thus be easily identified, e.g., by flow cytometry, and in particular cell fluorescence-activated cell sorting (FACS). Preferably, the endothelial cells do not express CD45.

Limited cell sources have been one of the major factors for failure of tissue-engineering approaches in the past (Ott et al., 2011). The present invention deliberately omits muscle cells from the Tunica media and provides a vein-like graft preferably consisting solely of endothelial cells and connective tissue. The endothelial cells used in the bio-inks of the invention can be derived from different sources. For example, the endothelial cells can be Human Umbilical Vein Endothelial Cells (HUVEC), mononuclear-derived endothelial-like cells, endothelial cells derived from induced pluripotent stem cells (iPS) and blood-derived endothelial cells. In a preferred embodiment, the endothelial cells are HUVEC. In another preferred embodiment, the endothelial cells are derived from mononuclear-derived endothelial-like cells. In yet another embodiment, the endothelial cells are derived from iPS. Preferably, the endothelial cells used in the bio-inks of the invention are human cells. In yet another embodiment, the endothelial cells are derived from punch biopsies.

Apart from or in addition to the differentiated endothelial cells, the bio-inks of the invention may also comprise cells which are capable of developing into endothelial cells. A preferred example of cells having the capacity to develop into endothelial cells are endothelial progenitor cells (EPCs). As used herein, the term "EPCs" refers to immature cells that have the capacity to differentiate into mature endothelial cells and express the cell surface markers CD31, CD34, CD146, CD309, while at the same time they do not express CD45 and CD14 (Mead et al., 2008; Emontzpohl et al., 2017). EPCs can be identified by morphological analysis and/or flow cytometry, and in particular FACS.

EPCs for use in the bio-ink of the invention have been extensively described in the prior art and can be readily obtained from whole blood samples as described in the literature before (Baudin et al. 2007, Mead et al., 2008). Briefly, mononuclear cells are isolated from blood using Ficoll density gradient centrifugation according to standard protocols, followed by washing with phosphate-buffered saline (PBS). Cells are cultured using endothelial basal medium 2 (EBM-2; Lonza, Basel, Switzerland) supplemented with EBM-2-MV-SingleQuots (Lonza, Basel, Switzerland). Cells in suspension are removed after 3 days of culture and adherent EPCs are passaged at 80-90% confluence (Mead et al., 2008; Emontzpohl et al., 2017; Kong et al., 2021). All cell cultivation steps are performed in a humidified atmosphere at 37°C and 5% $CO_2$. Apart from this manual procedure, EPCs can also be obtained via automated cell processing e.g., by using the CliniMACS Prodigy (Miltenyi) in combination with magnetic bead sorting.

SMCs can also be used in the bio-inks of the invention. These cells are regularly used in tissue engineering for the construction of vascular grafts. Preferably, the SMCs used in the bio-inks of the invention are of the contractile type which means that they are spindle-shaped cells which provide contractile filaments. SMCs can be obtained by differentiating autologous precursor cells from samples or by punch biopsy as described in Simper et al. 2002 or Kolster et al. 2017).

The number of cells that are added to the bio-ink composition of the invention may vary dependent on the graft to be produced. Typically, the bio-ink composition will comprise a total of $1 \times 10^3$ to $1 \times 10^8$ cells per ml, and preferably $1 \times 10^5$ to $1 \times 10^7$ cells per ml, and more preferably $1 \times 10^6$ to $5 \times 10^7$ cells per ml. In one embodiment, the bio-ink composition will comprise $1 \times 10^3$ to $1 \times 10^8$ endothelial cells per ml, and preferably $1 \times 10^5$ to $1 \times 10^7$ endothelial cells per ml, and more preferably $1 \times 10^6$ to $5 \times 10^7$ endothelial cells per ml. In another embodiment, the bio-ink composition will comprise $1 \times 10^3$ to $1 \times 10^8$ EPCs, and preferably $1 \times 10^5$ to $1 \times 10^7$ EPCs, and more preferably $1 \times 10^6$ to $5 \times 10^7$ EPCs. In yet another embodiment, the total number of endothelial cells and EPCs which are present in the bio-ink composition of the invention will be $1 \times 10^3$ to $1 \times 10^8$ cells, and preferably $1 \times 10^5$ to $1 \times 10^7$ cells, and more preferably $1 \times 10^6$ to $5 \times 10^7$ cells. In another embodiment, the bio-ink composition will comprise $1 \times 10^3$ to $1 \times 10^8$ SMCs, and preferably $1 \times 10^5$ to $1 \times 10^7$ SMCs, and more preferably $1 \times 10^6$ to $5 \times 10^7$ SMCs. According to the invention, the SMCs, endothelial cells and/or EPCs are mixed into the bio-ink composition of the invention. When printing the bio-ink composition, the cells become embedded into the graft, thereby providing stable biomechanical characteristics to the graft which distinguish the grafts of the invention from other preliminary approaches.

The bio-ink composition of the invention can be prepared by mixing the peptide-alginate complexes with an appropriate number of SMCs, endothelial cells and/or EPCs. Optionally, further excipient compounds may be added, e.g., compounds that are used to modulate the fluidity of the bio-ink to adapt it to a specific printing device. The aqueous bio-ink composition can then be cross-linked by the addition divalent and trivalent cations. For example, the bio-ink composition can be cross-linked by the addition $Ca^{2+}$, $Sr^{2+}$, $Ba^{2+}$, $Cu^{2+}$, $Zn^{2+}$, $Mn^{2+}$, $Fe^{2+}$, $Cr^{3+}$, or $Fe^{3+}$ cations. It is preferred herein to effect cross-linking by $Ca^{2+}$ cations. The cross-linking can be achieved during the printing process, preferably by mixing the aqueous bio-ink composition during the printing process with a solution containing the cation, e.g., a $CaCl_2$ solution. The mixing of the bio-ink composition can be carried out in the print head of the 3D printer.

Also provided is a method for manufacturing a bioartificial vascular graft in which the bio-ink composition of the invention is printed to a 3D bioartificial vascular graft by use of a 3D printer. The method comprises the following steps:

(a) loading a bio-ink composition according to the first aspect of the invention into a 3D printer which is capable of printing viscous hydrogels;

(b) contacting the bio-ink composition with a crosslinking solution comprising a divalent and/or trivalent cation in the 3D printer to induce crosslinking of the alginate;

(c) printing the bio-ink to obtain the bioartificial vascular graft.

In the first step of the method, a bio-ink composition as described herein above is loaded into a 3D printer which is capable of printing viscous hydrogels. The bio-ink composition preferably is an aqueous composition. For this purpose, a solution of an alginate is provided to which EM protein-derived peptides have been coupled. The amount of the alginate (including the peptide-grafted alginate) in the bio-ink composition can range from 1.0% to about 25.0% (w/w), based on the overall weight of the aqueous bio-ink composition. An amount of 5.0% (w/w) based on the overall weight of the aqueous bio-ink composition is particularly preferred.

Preferably, the amount of the alginate (including the peptide-grafted alginate) in the aqueous bio-ink composition will be about 2.0%, about 3.0%, about 4.0%, about 5.0%, about 6.0%, about 7.0%, about 8.0%, about 9.0%, about 10.0%, about 11.0%, about 12.0%, about 13.0%, about 14.0%, about 15.0% (w/w), about 16.0% (w/w), about 17.0% (w/w), about 18.0% (w/w), about 19.0% (w/w), about 20.0% (w/w), about 21.0% (w/w), about 22.0% (w/w), about 23.0% (w/w), about 24.0% (w/w), or about 25.0% (w/w), based on the overall weight of the aqueous bio-ink composition. In a particularly preferred embodiment, the amount of the alginate (peptide-grafted alginate) in the aqueous bio-ink composition is between 2.0 to 5.0%, and more preferably 4.0 to 5.0%, based on the overall weight of the aqueous bio-ink composition.

Then, the SMCs, endothelial cells and/or EPCs are added to the aqueous alginate solution. The cells are added in the numbers set forth above, i.e., a total of $1 \times 10^3$ to $1 \times 10^8$ cells per ml of the aqueous alginate solution, preferably $1 \times 10^6$

to $5 \times 10^7$.

In one embodiment, the amount of the alginate (including the peptide-grafted alginate) in the aqueous bio-ink composition is 2.0%, based on the overall weight of the aqueous bio-ink composition, and a total of $1 \times 10^3$ to $1 \times 10^8$ cells per ml of the aqueous alginate solution. In another embodiment, the amount of the alginate (including the peptide-grafted alginate) in the aqueous bio-ink composition is 3.0%, based on the overall weight of the aqueous bio-ink composition, and a total of $1 \times 10^3$ to $1 \times 10^8$ cells per ml of the aqueous alginate solution. In yet another embodiment, the amount of the alginate (including the peptide-grafted alginate) in the aqueous bio-ink composition is 4.0%, based on the overall weight of the aqueous bio-ink composition, and a total of $1 \times 10^3$ to $1 \times 10^8$ cells per ml of the aqueous alginate solution. In yet another embodiment, the amount of the alginate (including the peptide-grafted alginate) in the aqueous bio-ink composition is 5.0%, based on the overall weight of the aqueous bio-ink composition, and a total of $1 \times 10^3$ to $1 \times 10^8$ cells per ml of the aqueous alginate solution. In yet another embodiment, the amount of the alginate (including peptide-grafted alginate) in the aqueous bio-ink composition is 6.0%, based on the overall weight of the aqueous bio-ink composition, and a total of $1 \times 10^3$ to $1 \times 10^8$ cells per ml of the aqueous alginate solution.

In one embodiment, the amount of the alginate (including the peptide-grafted alginate) in the aqueous bio-ink composition is 2.0%, based on the overall weight of the aqueous bio-ink composition, and a total of $1 \times 10^6$ to $5 \times 10^7$ cells per ml of the aqueous alginate solution. In another embodiment, the amount of the alginate (including the peptide-grafted alginate) in the aqueous bio-ink composition is 3.0%, based on the overall weight of the aqueous bio-ink composition, and a total of $1 \times 10^6$ to $5 \times 10^7$ cells per ml of the aqueous alginate solution. In yet another embodiment, the amount of the alginate (including the peptide-grafted alginate) in the aqueous bio-ink composition is 4.0%, based on the overall weight of the aqueous bio-ink composition, and a total of $1 \times 10^6$ to $5 \times 10^7$ cells per ml of the aqueous alginate solution. In yet another embodiment, the amount of the alginate (including the peptide-grafted alginate) in the aqueous bio-ink composition is 5.0%, based on the overall weight of the aqueous bio-ink composition, and a total of $1 \times 10^6$ to $5 \times 10^7$ cells per ml of the aqueous alginate solution. In yet another embodiment, the amount of the alginate (including the peptide-grafted alginate) in the aqueous bio-ink composition is 6.0%, based on the overall weight of the aqueous bio-ink composition, and a total of $1 \times 10^6$ to $5 \times 10^7$ cells per ml of the aqueous alginate solution.

The aqueous bio-ink composition of the invention can be reconstituted from a freeze-dried alginate-peptide product by adding distilled water or suitable buffer or a medium.

In the second step of the method, the bio-ink composition is contacted with a crosslinking solution comprising a divalent and/or trivalent cation in the 3D printer to induce crosslinking of the alginate. Preferably, the bio-ink composition is contacted with a solution comprising $Ca^{2+}$, $Sr^{2+}$, $Ba^{2+}$, $Cu^{2+}$, $Zn^{2+}$, $Mn^{2+}$, $Fe^{2+}$, $Cr^{3+}$, or $Fe^{3+}$ cations. It is preferred herein to effect cross-linking by $Ca^{2+}$ cations. Preferably, the cross-linking is achieved during the printing process by contacting the aqueous bio-ink composition with a solution containing the cation, e.g., a 2%, 3% or 4% $CaCl_2$ solution.

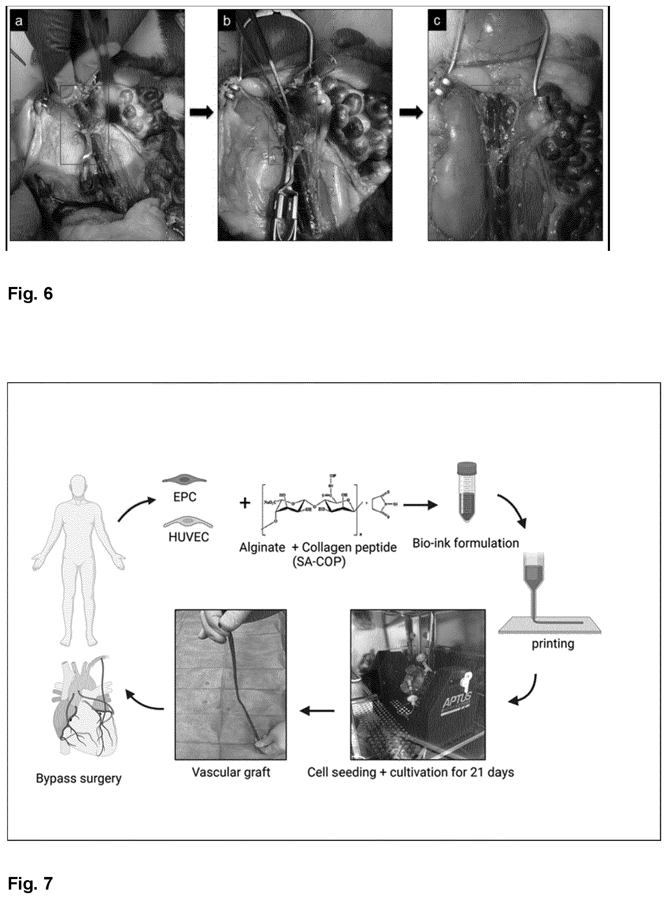

The contacting of the bio-ink composition and the crosslinking solution can be carried out in the print head of the 3D printer. In principle, any type of 3D printer is suitable provided that viscous hydrogels can be printed with the device. A suitable printer preferably comprises a print head with parametric flexibility that allows seamless adjustment of graft proportions without remodeling. The print head itself can be manufactured by 3D-printing using a clear resin. The print head is preferably designed to comprise a co-axial print head and two chambers, a first inflow chamber for the crosslinking solution and a second inflow chamber for the bio-ink composition. The diameter of the first inflow chamber is between 1.5-10 mm, and preferably between 2.5 mm and 3.5 mm, and the diameter of the second inflow chamber is between 1.5 mm and 2.5 mm, and preferably between 1.5 mm and 2.0 mm. In one embodiment, each of the two chambers are equipped with a Luer-Lock connector as an inlet and two nested tubes as outlets. Before the outlet, each chamber has a constriction serving as a resistance. The resulting congestion homogenizes the flow of the bio-ink and ensures a uniform wall thickness. Additionally, immediately before the resistance, radially arranged lamellae are present, aligning the flow longitudinally. A suitable print head is exemplified in Figures 7a and 7b. The average extrusion pressure is preferably set at 50-150 kPa, and preferably at 80-120 kPa, more preferably 100 kPa. The printing extrusion volume is preferably set at 5-50 ml/min, and preferably at 10-30 kPa, more preferably 20 ml/min.

In the last step of the method, the bio-ink is printed in the 3D printer into the bioartificial vascular graft. The printer that is used for carrying out the process preferably is a printer that is capable of printing viscous fluids or hydrogels. Such a printer has a high extrusion power of The average extrusion pressure is preferably set at 150 kPa or more, and a co-axial print head suitable for extruding viscous fluids or hydrogels. Additionally, such printer preferably provides adequate cubic capacity for printing a graft of 30-40 cm length or longer with precise industrial standard proceeding with a repeat accuracy of $\pm$ 0.2 mm. The printer preferably is suitable of being used in in a clean room facility under GMP standards.

The printing process can be carried out fully-automated without any manual intervention. Preferably, the printing process is performed in a sterile environment to provide GMP-compliant conditions. The bioartificial vascular graft printed in this way can have a length of up to 100 cm length. In a preferred embodiment, the bioartificial vascular graft has a length between 10 cm and 100 cm, and more preferably between 10 cm and 90 cm, between 10 cm and 80 cm, between 10 cm and 70 cm, between 10 cm and 60 cm, between 10 cm and 50 cm, between 10 cm and 40 cm, between 10 cm and 30 cm, or between 10 cm and 20 cm. A bioartificial vascular graft having a length between 10 cm and 30 cm is particularly preferred herein. Moreover, branched vascular grafts can be fabricated as well by the method of the invention, e.g. by using printer with a print head with a built-in branch or junction.

The method of the invention for preparing a bioartificial vascular graft may further comprise a step (d) which comprises incubating the printed bioartificial vascular graft obtained from step (c) in a solution comprising a divalent and/or trivalent cation. This step may be helpful for achieving a complete alginate crosslinking. For this purpose, the bioartificial vascular graft is immersed in a solution comprising the divalent and/or trivalent cation. The nature and concentration of the cation solution used in this step can be as described above. Preferably, a 2%, 3% or 4% $CaCl_2$ solution can be used.

The method of the invention for preparing a bioartificial vascular graft may also comprise a step (e) which comprises the further culturing of the graft obtained from step (c) or (d) in a bioreactor to provide the complete differentiation of the cells on the grafts. Therefore, in a preferred embodiment, the bioartificial vascular graft obtained from step (c) or (d) is transferred into a bioreactor for cell culturing. A standard culturing medium can be used, such as Endothelial Cell Growth Medium (ECGM, PromoCell, Heidelberg, Germany) which is supplemented with 4 $\mu$L/mL of endothelial cell growth supplement, 0.1 ng/mL epidermal growth factor, 1 ng/mL basic fibroblast growth factor, 90 $\mu$g/mL heparin, 1 $\mu$g/mL hydrocortisone (all from PromoCell) and 10% fetal bovine serum (Thermo Fisher, Dreieich, Germany). To comply GMP conditions, human AB serum can be used in a concentration of 2-10% instead of bovine serum. Another medium which can be used is the RPMI 1640 culture medium which is supplemented with 10% human serum. DMEM/Ham's 12 medium which is supplemented with 10% human serum can also be used. Preferably, the cell culturing is performed for 12 hours, 24 hours, 36 hours, 48 hours, 60 hours, 72 hours, or more. More preferably, the cell culturing is performed for at least 10 days, 14 days, 21 days, or longer. The cell culturing is preferably continued until a minimum cell number of about $1.0 \times 10^6$ cells/cm$^3$, about $1.2 \times 10^6$ cells/cm$^3$ or about $1.5 \times 10^6$ cells/cm$^3$ is achieved. Cell culturing is preferably performed under humidified conditions at 37°C and 5% $CO_2$.

The invention also provides a bioartificial vascular graft obtainable by the above method. The bioartificial vascular graft preferably is a tube or tube-like structure with a length as indicated above and with a minimum cell number of about $1.0 \times 10^6$ cells/cm$^3$, about $1.2 \times 10^6$ cells/cm$^3$ or about $1.5 \times 10^6$ cells/cm$^3$. Preferably, the graft has a mean total diameter of 2,000-4,000 $\mu$m, and more preferably 2,500-3,500 $\mu$m, and an average wall thickness of 200-400 $\mu$m, and more preferably 250-350 $\mu$m, which corresponds to average dimensions of small peripheral arteries and veins.

The printed bioartificial vascular grafts can be used for a variety of medicinal and non-medicinal purposes, including therapy, diagnostics, and drug screening. In addition, the printed bioartificial vascular grafts can be used as an in-vitro training model for endovascular or surgical training.

The invention hence provides a bioartificial vascular graft as described above for use in medicine. In particular, the bioartificial vascular graft is for use in vascular or cardiovascular therapy. For example, the printed bioartificial vascular grafts can be therapeutically used, e.g., for vascular or cardiovascular bypass surgery, for vascular or cardiovascular vessel repair, or for the preparation of a vascular or cardiovascular conduit after vessel trauma.

The printed bioartificial vascular grafts can also be used as an in-vitro training model for endovascular or surgical training. The graft-based training model can be used for suturing, surgical preparation as well as catheter-based training. In addition, hematological and vessel-related diseases can be modelled and analysed with the printed bioartificial vascular grafts of the invention as an in-vitro model or in a lab-on-a-chip approach.

The printed bioartificial grafts can also be used for the analysis of hematological and vessel-related diseases and for in vitro drug screening.

## BRIEF DESCRIPTION OF THE FIGURES

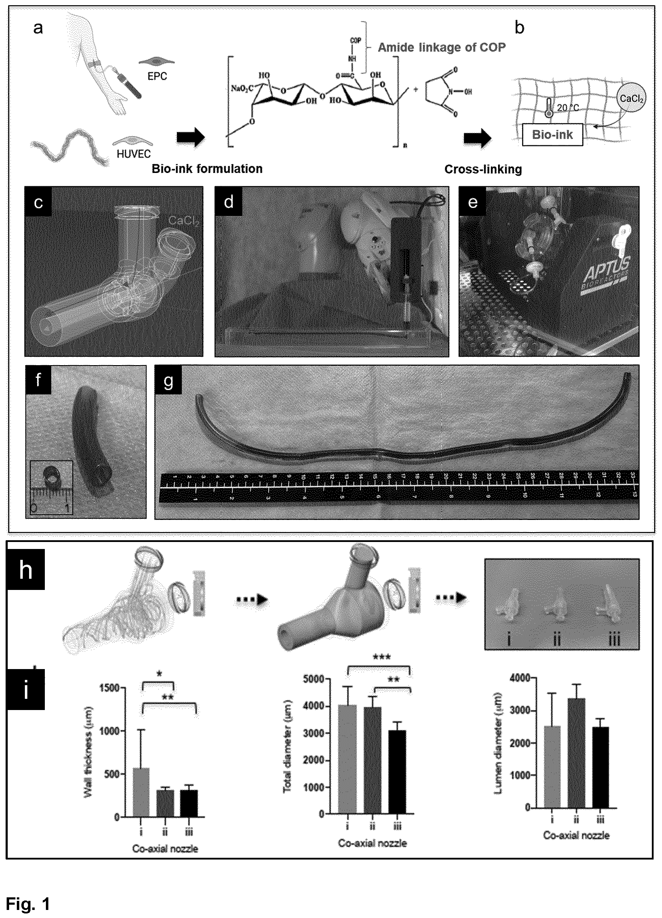

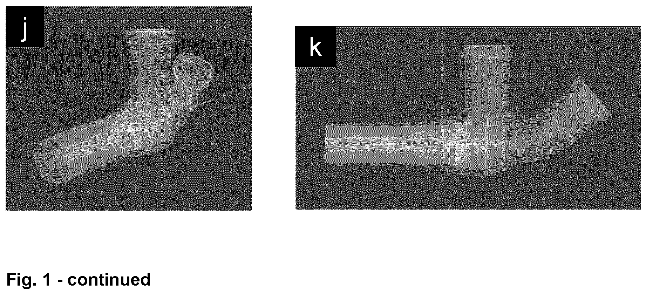

Figure 1 shows the fabrication of the vascular grafts and design of the 3D bioprinting process. a) Endothelial Progenitor Cells (EPC) or Human Umbilical Vein Endothelial Cells (HUVEC) respectively are integrated into Collagen Peptide grafted Sodium Alginate (SA-COP) for the fabrication of the bio-ink. b) Immediate cross-linking of SA-COP by $CaCl_2$ is conducted via the co-axial printing process into support medium. c + d) 3D bioprinting is performed by the customized printing platform with an extrusion-based, co-axial print head. e) After bioprinting, the grafts are transferred to the bioreactor. f) Final design of the ready-to-implant vascular grafts providing a vessel lumen between 3-3.5 mm and g) a total length of 30-40 cm. h) Flow and printing simulation of the print head were performed by the Ansys simulation software to create the print head prototypes i-iii. Print head iii represents the blueprint of the final print head design and enables the printing of the finalized bioartificial graft composition (BG$_{SA-COP20\%}$). i) The reproducibility and holistic structure of vessel wall thickness, total diameter and lumen diameter were consistently refined during the simulation of the bioprinting process as shown by the performance results of the print head prototypes i-iii. Schematic illustration of the parametric print head used in the present examples, j) Schematic overview of the parametric print head. k) Sideview of the parametric print head.

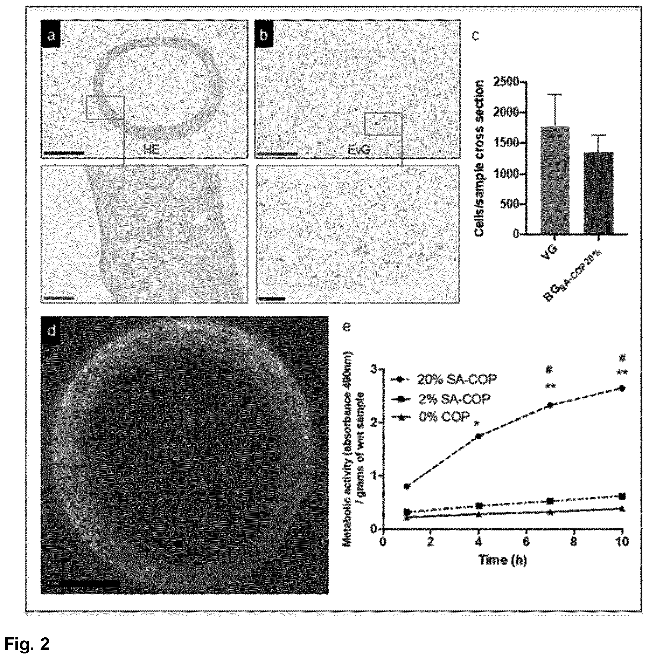

Figure 2 shows the histological and immunohistological characterization of the 3D printed bioartificial grafts (BG$_{SA-COP20\%}$). a) Representative complete bypass cross section (HE staining) with magnification (red rectangle). b) Elastica van Gieson (EvG) staining with magnification (red rectangle). c) Cell number per cross section in human Vein Grafts (VG) compared to the cell number per cross section in the printed bioartificial grafts (BG$_{SA-COP20\%}$). d) 3D life imaging via fluorometric visualization with Calcein staining after 21 days of cultivation. e) MTS absorbance (metabolic activity) of wet sample of Bioartificial Grafts from Sodium Alginate ($BG_{SA}$), Bioartificial Grafts from Collagen Peptides grafted Sodium Alginate 2% ($BG_{SA-COP2\%}$) and 20% ($BG_{SA-COP20\%}$); *** = $p < 0.001$.

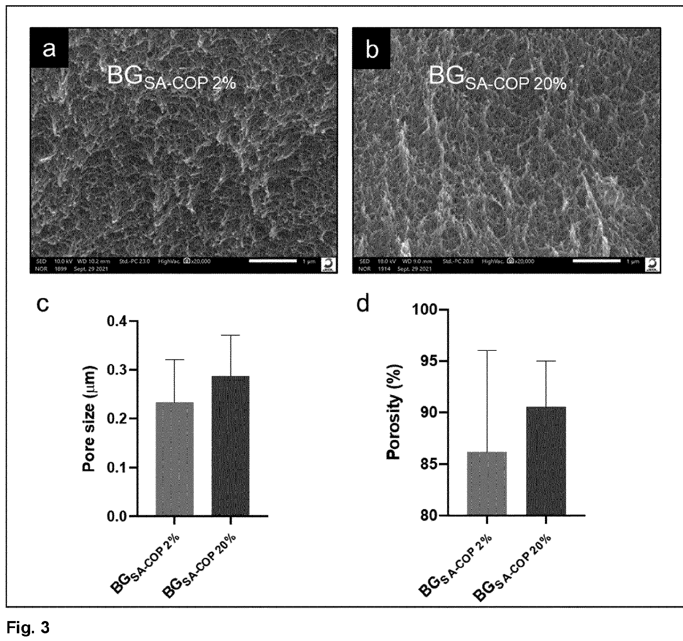

Figure 3 Ultrastructural presentation of the Bioartificial Graft(s) from Collagen Peptides grafted Sodium Alginate ($BG_{SA-COP}$). a + b) Surface of the bioartificial grafts and depiction of the fine network of interconnected fibers of $BG_{SA-COP}$. c) Comparison of scaffold pore size ($\mu$m) between $BG_{SA-COP\ 2\%}$ and $BG_{SA-COP\ 20\%}$. d) Comparison of porosity (%) between $BG_{SA-COP\ 2\%}$ and $BG_{SA-COP\ 20\%}$.

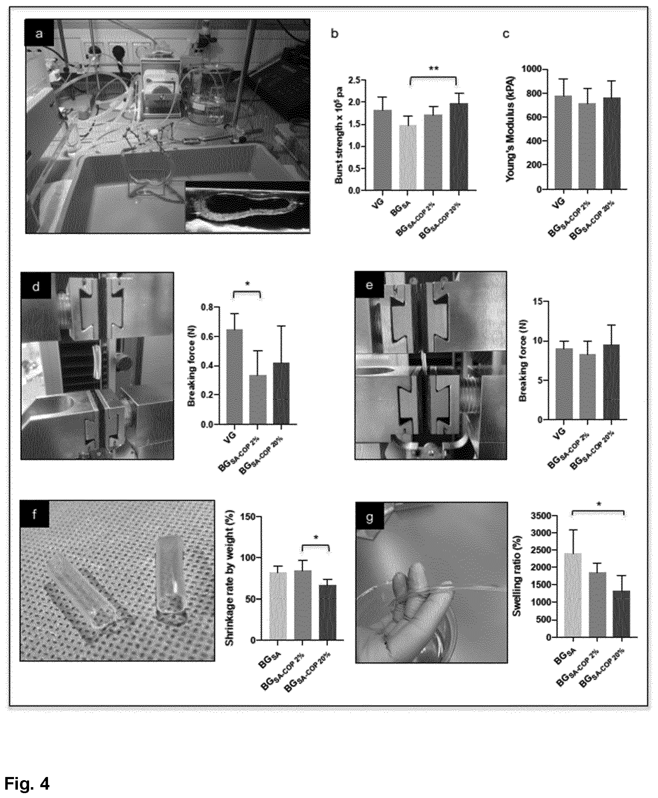

Figure 4 shows the results of the comparable biomechanical testing of human Vein Grafts (VG), Bioartificial Grafts from Sodium Alginate (BG-SA) and Bioartificial Grafts from Collagen Peptides grafted Sodium Alginate (BG-SA-COP). a) Simulation of small-vessel perfusion, burst strength and long-term resilience of BG-SA-COP in a flow model and sonographic control of the grafts (red rectangle). b + c) Burst strength of VG, BG-SA and BG-SA-COP and Young's Modulus of VG and BG-SA-COP. d + e) Suture and tensile strength of VG and BG-SA-COP. f + g) Shrinkage Rate by Weight (SRW) and Swelling Ratio (SR) of BG-SA and BG-SA-COP; * = $p < 0.05$; ** = $p < 0.01$.

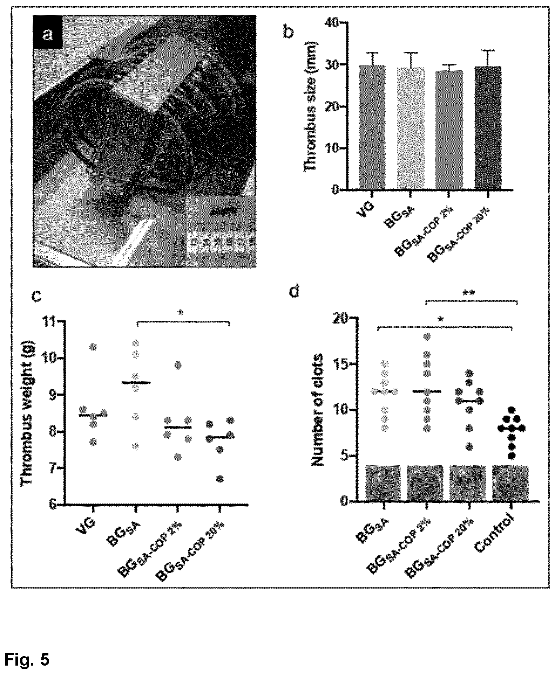

Figure 5 shows the results of the assessment of coagulation and platelet activation. a) Chandler Loop model and exemplary depiction of thrombus (red rectangle). b.) Comprehensive analysis of thrombus size between human Vein Grafts (VG), Bioartificial Grafts from Sodium Alginate (BG-SA) and Bioartificial Grafts from Collagen Peptides grafted Sodium Alginate (BG-SA-COP) after 60 min of circulation in the Chandler Loop. c) Comprehensive analysis of thrombus weight between VG, BG-SA and BG-SA-COP after 60 min of circulation in the Chandler Loop. d) Total number of clots in the platelet-activation-assay after 15 min; * = $p < 0.05$; ** = $p < 0.01$; surface of empty well was used as a control.

Figure 6 shows the surgical implantation of the 3D printed Bioartificial Grafts from Sodium Alginate 20% ($BG_{SA-COP20\%}$) in a cadaver model (red rectangle a-c). a) Parachute anastomotic technique was performed for cardiovascular graft implantation. b) Pull-down maneuver of the bioprinted vascular graft and c) final suturing of the graft creating a sufficient anastomosis in the rabbit aorta.

Figure 7 shows an overview on the novel 3D bioprinting technique of the invention.

## EXAMPLES

The examples described below were approved by the local ethics committee of the University Medical Center Schleswig-Holstein, Kiel, Germany (protocol identification number: D518/13, D513/19 and D451/21 respectively). All procedures were performed in accordance with the Helsinki Declaration of 2013.

Experimental groups comprised human saphenous Vein Grafts (VG), Bioartificial Grafts from Sodium Alginate (BG-SA), Bioartificial Grafts from Collagen Peptides grafted Sodium Alginate 2% and 20% (BG-SA-COP-2 and BG-SA-COP-20). VG were obtained from voluntary donors during cardiac bypass surgery. The tissue quality of the VG and suitability for cardiovascular bypass grafting was assessed by duplex sonography and two cardiovascular surgeons not involved in the here described proof-of-concept study. Vascular grafts for the biomechanical experiments had a standard length of 60 mm and a standard diameter of 3.0-4.5 mm.

Each experiment was repeated three times if not stated otherwise. All data are presented by the Mean $\pm$ Standard Deviation (SD). Categorical variables are presented as frequency distributions (n) and percentage (%). Data were analysed by using Graph Pad Prism version 9.2.0 (Graph Pad Software; San Diego, USA). Sample size calculation was performed by the free available G*power Software and StatMate (Graph Pad Software; San Diego, USA). Normality was tested by using the Shapiro-Wilk and Kolmogorov-Smirnov test. Data were analysed using one- and two-way ANOVA, Kruskal-Wallis-Test, or Student's t test if appropriate. Tukey's multiple comparison was performed as post-hoc-test. A p-value < 0.05 was considered as significant.

Example 1: Preparation of a bio-ink for 3D bioprinting

A bio-ink was prepared by crosslinking a synthetic collagen-derived peptide (COP) that resembles the primary structure of collagen (N-(3-[2-Furyl]acryloyl)-L-G-P-A, available as FALGPA from SIGMA-Aldrich), and sodium alginate (SA) using the carbodiimide method. 0.35 g SA was dissolved in 29 ml 0.2 M MES (2-(N-morpholino) ethanesulfonic acid) buffer containing 0.3 M NaCl, pH 5.5. Then, 0.035 g N-hydroxy sulfosuccinimide (NHS) and 0.035 g EDAC (1-Ethyl-3-(3'-dimethylaminopropyl-carbodiimide, HCl) were added step by step into the solution at room temperature with magnetic stirring for 20 min. The pH was slowly increased to 7.0 and the solution was set to a final volume of 35 ml (1% alginate solution). For preparing a bio-ink composition with 2% of COP coupled to SA ($BG_{SA-COP2\%}$) 3.5 mg of the FALGPA peptide was added into the solution. In a parallel approach, 35 mg of the FALGPA peptide was added into the solution to prepare a bio-ink composition with 20% of COP coupled to SA ($BG_{SA-COP20\%}$). The coupling reaction was allowed to proceed for 7-8 h under stirring.

The two solutions so produced were purified by dialysis through a 6-8 KD molecular weight cut-off dialysis tube for three days. The dialyzed products were finally freeze-dried with LyoVac GT2 freeze dryer to obtain purified alginate derivative powders. The dried samples were stored at -20°C. Freeze-dried alginate products without and with coupled collagen peptide were reconstituted to 4% alginate with sterile distilled water. The coupling of peptide to alginate was confirmed by measuring the differences in the absorbances compared to an alginate control. The increase in absorbance by the peptide was calculated as followed:

$$\text{Increase of absorbance (\%)} = [\text{Abs (SA-COP)} - \text{Abs (SA)}] / \text{Abs (SA)} \times 100$$

The presence of peptide increases the absorption of UV light at 200 nm (for peptide bonds), at 280 nm (for aromatic rings) and at 345 nm (for FALGPA) as described previously (Jackson et al., 1995; Morch et al., 2007).

Results: Different bio-ink compositions were produced. Specifically, bio-ink compositions with low (2%) and high concentration (20%) of COP coupled to SA were used for the bioprinting of bioartificial grafts ($BG_{SA-COP2\%}$, $BG_{SA-COP20\%}$) and compared to sole SA manufactured grafts (BG-SA) and human Vein Grafts (VG) to investigate the biomimetic characteristics.

Example 2: Preparation of a cell-containing bio-ink

The final bio-ink for use in 3D bioprinting was prepared by adding Human Umbilical Vein Endothelial Cells (HUVEC) or Endothelial Progenitor Cells (EPC) to the matrix obtained from Example 1. HUVEC were isolated from umbilical cords and cultured in endothelial cell growth medium ECGM (PromoCell, Heidelberg, Germany) supplemented with 4 µL/mL of endothelial cell growth supplement, 0.1 ng/mL epidermal growth factor, 1 ng/mL basic fibroblast growth factor, 90 µg/mL heparin, 1 µg/mL hydrocortisone (all from PromoCell) and 10% fetal bovine serum (Thermo Fisher, Waltham, USA) (Baudin et al. 2007). A suitable protocol for the isolation of HUVEC is described in (Crampton et al. 2007). For complying with GMP conditions, the bovine serum can be replaced by 2-10% human AB serum.

EPC were obtained from whole blood samples (40 ml) of healthy volunteers after informed consent and in accordance with the local ethics committee (see the protocol in: Mead et al., 2008). Briefly, whole blood was mixed Mix 1:1 with $Ca^{2+}$-$Mg^{2+}$-free PBS and blood mononuclear cells were isolated using Ficoll density gradient centrifugation according to the manufacturer's protocol (GE Healthcare, Chicago, USA), followed by washing with phosphate-buffered saline (PBS). Further isolation of EPC was performed by CD34+ cell-sorting via magnetic beads according to the manufacturer's protocol (Miltenyi, Bergisch Gladbach, Germany). Cells were then plated in collagen-coated flasks and cultured with endothelial basal medium 2 (EBM-2; Lonza, Basel, Switzerland). Characterization of EPC was performed by morphological analysis and flow cytometry. Briefly, flow cytometry was performed using the BD FACS Calibur™ cytometer (BD Biosciences, Franklin Lakes, USA). Specific antibodies and their corresponding isotypes (dilution for all antibodies: 1:20) were directly conjugated with fluorescein isothiocyanate (FITC), alexa fluor, or allophycocyanin (APC)-Cy7. The gating strategy consisted of (i) identification of EPC based on their size and granularity (FSC vs SSC profiles), (ii) exclusion of non-viable cells and (iii) identification of EPC positive for CD31, CD34, CD146, CD309 and negative of CD45 and CD14.

HUVEC or EPC were detached, counted and maintained in cell culture medium before preparation of the final bio-ink formulation. The desired calculated cell quantity was carefully mixed with the 4% SA-COP solutions to obtain final cell concentration in the bio-ink of $2.5 \times 10^6$ cells/ml and 3% of SA-COP in the final bio-ink.

Results: The bio-ink comprising SA-COP and the EPCs resembled the physical composition of a viscous gel at room temperature.

Example 3: 3D bioprinting of vessel-like structure

Since most of the available commercial 3D bioprinting systems do not provide adequate cubic capacity for printing a vascular graft of 30-40 cm length (Figure 1f + g), a customized 3D bioprinting platform was used in order to obtain a homogenic outflow in axial direction for the extrusion of the high-viscosity bio-ink (Figure 1c + d). The Ansys CFX 2020 R2 simulation software (Ansys, Canonsburg, USA) was applied for prototyping of the print head design (Figure 1h + i; print head prototype i-iii) and the simulation of the printing process itself.

Viscosity was set at 8.3 Pa*s and density was set at 1.79 g·cm$^{-3}$ for alginate during the simulation process. The final print head design (Figure 1h + i; print head iii) included a main inflow chamber for the CaCl$_2$ support medium for immediate cross-linking and a lateral inflow chamber running the cell-loaded bio-ink (Figure 1c). It is commonly accepted that immediate and rapid cross-linking of microfibers during the printing process is mandatory for the later resilience of the fabricated grafts. The central inflow chamber conducted the 4% CaCl$_2$ support medium for rapid cross-linking and the lateral inflow chamber provided the cell-loaded bio-ink with an average flow of 20 ml/min (Figure 1c). The printing process was performed at room temperature, pressure-controlled and extrusion-based due to the high viscosity of the bio-ink. Average extrusion pressure was set at 100 kPa with a printing speed of 20 ml/min. The co-axial print head was guided by a 6-axis robot arm (Stäubli TX2-40 6-axis robot; Stäubli, Pfäffikon, Switzerland) enabling precise proceeding with a repeat accuracy of +/- 0.2 mm, six degrees of freedom and a sterile printing process without potential contamination.

The BGs were printed into support medium of 4% $CaCl_2$ for additional cross-linking (Figure 1d).

<u>Results:</u> Advanced simulation of the in- and outflow characteristics of the co-axial printing process significantly improved and homogenized graft morphology (Figure 1h + i + j + k). Reproducibility of the bioprinting process improved significantly after computational flow simulation and was accordingly high in the final configuration (e.g., standard error of wall thickness of the vascular grafts < 10%). The final BGs had a mean total diameter of 3092 $\pm$ 307.7 $\mu$m, a lumen of 2472 $\pm$ 275.6 $\mu$m and average wall thickness of 307.2 $\pm$ 67.97 $\mu$m which is in line with average dimensions of small peripheral arteries and veins. The final 3D bioprinting platform enabled the fabrication of a BG with a length of 30-40 cm which is comparable with a human saphenous vein from the lower or upper leg commonly used as a VG for cardiovascular surgery (Majesky et al., 2018; Kentenciller et al., 2018) (Figure 1f + g). These data clearly suggest that the grafts prepared according to the invention are suitable for use in human medicine (Figure 1 + 2).

The crosslinking of the bio-ink by $CaCl_2$ leads to a shrinkage of the bypass graft during the manufacturing process. Additionally, the bio-ink exhibits non-Newtonian fluid behavior. Both factors necessitate the incorporation of an offset into the parameters for the diameter and wall thickness of the grafts to achieve the target parameters. The size proportions of the produced graft were carefully measured under a microscope and compared to the target parameters. Over three iterations, an optimal offset for the diameter and wall thickness parameters was determined (Figure 1j +k). The following Table 1 presents the results for the graft and print head parameters used in this study:

**Table 1:** Results for the graft and print head parameters used in this study.

|  | Target parameter | Print head parameter |
|---|---|---|
| Diameter | 4 mm | 5.2 mm |
| Wall thickness | 0.8 mm | 1.5 mm |
| Lumen | 2.4 mm | 2.2 mm |

<u>Example</u> 4: Cultivation of the vascular grafts in the bioreactor

After printing, the vascular grafts remained 6 h in 4% $CaCl_2$ and culture medium (1:1) and were then cultivated for 72 h in a rotating cell seeder (Aptus Bioreactors, Clemson, USA), a bioreactor (Figure 1e) with a slightly modified protocol (Movileanu et al., 2021). Briefly, the bioreactor settings were chosen by experimental pre-testing to enable a continuous flow during reactor movement, regular rest periods for advanced cell encapsulation and prevention of exceeded wall-attachment. After a period of static immersion in culture media for 1 h the bioreactor was rotated at 40° and 60° respectively and at 3 rotations per minute (RPM). There was a programmed rest period for 10 min after each cycle. For this dynamic cell seeding, an air pump was attached to the rotator jars via a sterile inline filter, continuously enriching the culture media with air from the $CO_2$ incubator (Movileanu et al., 2021). After the cell seeding, the grafts were transferred to conventional 3D cell culture. The vascular grafts were cultured for a total of 21 days in order to reach a minimum estimated cell number of approximately $1.6 \times 10^6$ cells/cm$^3$. Cultivation was performed under humidified conditions at 37°C and 5% $CO_2$.

<u>Example 5</u>: Analysis of cell viability, density, and proliferation

The cultured grafts obtained from Example 4 were analyzed by fluorometric visualization to determine cell viability, density and proliferation after 21 days of cultivation. Living cells were qualitatively assessed by fluorometric visualization. Small pieces (about 0.5 cm) of the bio-ink were removed and added in another well with fresh medium containing 0.1 $\mu$M Calcein AM (Thermo Fisher, Waltham, USA) and 2 $\mu$g/ml Hoechst 33342 (Invitrogen, Carlsbad, USA) for the assessment of cell viability and cell nuclei, respectively. Staining of cells was left for 30 minutes inside of the incubator in the dark. A small piece of bio-ink was mounted between a glass slide and a coverslip. Live cells were visualized using a Leica microscope (Leica DM2000, Leica, Wetzlar, Germany) with fluorescent filters. Cell numbers and densities (nuclei/cm$^2$) were evaluated in fluorescent images of Hoechst 33342-stained cells using the automatic image-based tool for counting nuclei (ITCN) from the Image J software 1.41 (NIH).

The metabolic activity and cell proliferation within the vascular graft were evaluated after 21 days of cultivation by using a colorimetric kit (CellTiter96® AQueous One Solution Reagent G3580 Promega Madison, WI, USA). Small specimens (about 1 cm in length) of the vascular graft were removed and added into a new culture plate with 1 ml of fresh culture medium. 200 $\mu$l of MTS reagent was applied to each well and culture was continued at 37°C. Metabolic

active cells release a colored product into the culture medium that was evaluated by absorbance at 490 nm using an ELISA reader (Tecan, Crailsheim, Austria) in combination with the Magellan software v1.1. Every hour between 1-10 hours of incubation after the addition of MTS reagent, 100$\mu$l of culture media was taken at each time point and analyzed. At the end of the experiment, the BG specimen used to evaluate metabolic activity by MTS was removed, let dry completely at 95°C for 48h and weighed. For the graphic, the absorbance per gram of wet sample was depicted at each time point. The metabolic activity per sample at each time point was calculated as follow:

$$\text{Metabolic activity: (MTS Abs}_{490nm} \text{ / grams of wet sample)}$$

Samples of the vascular grafts were furthermore visualized by Live-3D-Cell-Imaging using a Leica THUNDER Imager 3D assay (Leica Microsystems GmbH, Wetzlar, Germany) based on Leica DMi8 microscope with 5x objective lens (PLAN 5x/0.12 dry), 20x objective lens (HC PL APO 20x /0.80 dry) and Leica K5 camera (4.2 MP, 40 FPS). Cool LED pE4000 was used as a fluorescence light source and the filter cubes with the desired excitation and emissions were selected (DAPI or LED-405 EX: 405/60, EM: 470/40; or FITC EX:480/40, EM:527/30 or TXR EX:560/40, EM:630/75). Normal or top to bottom Z-stack images of the samples were acquired using the same set of filters and settings (intensity, exposure time, threshold and Z-step size). Images were processed with Leica LAS X software and the Small Volume Computational Clearing (SVCC) was applied. Immunofluorescence staining was analysed using the Image J software 1.41 (National Institutes of Health NIH) and cell number in 3D cell culture was analysed layer-by-layer. Total cell number for the whole vascular grafts was finally calculated based on a thickness of 4 $\mu$m for every specimen and a total of 15000 layers within the cultured vascular grafts respectively. Specimen of human native veins served as control group.

Samples of the vascular grafts were also fixed in 4% formalin and embedded in paraffin. Six 4 $\mu$m thick sections from the proximal, middle and distal end were prepared and routinely stained with Haematoxylin-Eosin (HE), Elastica van Gieson (EvG) and Sirius Red. Immunohistochemical CD31 staining was performed with an automated Bondmax staining system (Leica Biosystems, Wetzlar, Germany) using the Polymer Refine Detection Kit (Leica Biosystems) and anti-CD31 antibody (clone JC70, Cell Marque, Rocklin, California, United States) at a dilution of 1:100.

Results: After initial bioprinting and cultivation in the bioreactor, cell density, viability and cell numbers were qualitatively assessed by fluorometric visualization (Figure 2d). Excellent viability and significant higher cell density could be demonstrated in SA-COP in contrast to SA. After 21 days in culture, endothelial cells within the bioartificial vessel wall showed continuous metabolic and a significant higher proliferative activity in BG$_{SA-COP20\%}$ (Figure 2e). It is well-known that alginate does not provide any cell-binding motifs in contrast to collagen. Thus, the increase of the proportion of collagen in SA-COP hydrogels enables significant more cell adhesion and thereby maintains cell viability and proliferation. It is also likely that COP could stimulate the synthesis of extracellular matrix components and consequently speed up tissue formation and promote cell adhesion and proliferation (Fan et al., 2013; Ullah et al., 2020; Hu et al., 2021).

Further immunobiological staining of the BG-SA-COP demonstrated strong expression of the endothelial surface marker CD31. Collagen-specific analyses employing Elastica von Gieson (EvG) and Sirius Red staining confirmed the presence of a dense matrix (Figure 2b). After a cultivation period of 21 days, Calcein AM staining revealed high cell viability and a tendency for cellular organization in the evolved graft as demonstrated by Live-3D-Cell-Imaging (Figure 2d). Total cell number in specimens of BG-SA-COP was slightly lower than in control samples of VG but without statistical significance (Figure 2c). Calculation of the total number of viable cells per vascular graft yielded approximately $2.1 \times 10^7$ cells and was comparable to specimens of VG with an estimated total cell number of $2.6 \times 10^7$ (graft dimensions: 60 mm x 3.0 - 4.5 mm).

Taken together, advanced morphological examination of the BG$_{SA-COP20\%}$ revealed a tendency of cellular organization and homogenous artificial tissue growth within a collagen-based matrix which suggest biomimetic characteristics of the fabricated vascular grafts.

Example 6: Analysis of porosity and pore size

The cultured grafts obtained from Example 4 were further analyzed by Scanning Electron Microscopy (SEM) to determine pore size and tissue network of the vascular grafts. Vessel specimens were fixed in 2.5% glutaraldehyde and postfixed in 2% osmium tetroxide, irrigated with distilled water and then dehydrated in series of 50-100% ethanol. The specimens were then placed in 100% ethanol and dried by critical point dryer (Balzers, CPD030) and mounted onto SEM slabs and sputter-coated for 50 seconds, 38mA with gold by sputter coater

(Balzers, SCD050). Examination of the vessel specimens was performed with a JSM-IT200 (JEOL, Tokyo). The Image J software was used to analyze SEM images to assess mean pore sizes and to establish pore size distributions in the samples. Porosity was measured via a liquid substitution approach using the following formula and as described before (Xu et al., 2021): p (%) = (V1-V3)/(V2-V3), where p corresponds to porosity, V1 denotes the starting volume of ethanol, V2 denotes the volume of ethanol following sample immersion for 10 min, and V3 denotes the residual volume following wet sample removal.

Results: Scanning Electron Microscopy (SEM) imaging revealed a porous and well-interconnected microstructure as seen in Figure 3a + b. Pore size for embedded cells or cell debris ranged from 10.40-81.50 $\mu$m in BG$_{SA-COP 2\%}$ and 9.08-78.30 $\mu$m in BG$_{SA-COP 20\%}$ and from 0.11-0.42 $\mu$m in BG$_{SA-COP 2\%}$ and 0.17-0.43 $\mu$m in BG$_{SA-COP 20\%}$ for the surface of the bioartificial vessel wall (Figure 3c). Both BG$_{SA-COP}$ were highly porous with porosity values as demonstrated in Figure 3d. As described before the degree of porosity and refining is crucial for integration of biomaterials determining cell seeding, proliferation and cell metabolism [25, 26, 27]. As described before the degree of porosity and refining is crucial for integration of biomaterials determining future cell seeding, proliferation and cell metabolism (Tamaddon et al., 2017; Xu et al., 2021; Su et al., 2021). High-porosity scaffolds (e.g. > 70% porosity) indicate good nutrition supply and neo-vascularization enabling stable long-term results after in-vivo implantation. Generally, a pore size range of 10-500 $\mu$m is considered as beneficial for scaffold integration which is in common with the here described experimental evaluation of both BG-SA-COP (Xu et al., 2021; Su et al., 2021).

Example 7: Analysis of biomechanical characteristics

The cultured grafts obtained from Example 4 were further analyzed for their biomechanical characteristics. Burst strength of VG, BG-SA, $BG_{SA-COP2\%}$ and $BG_{SA-COP20\%}$ were compared using a previously described method (Clements et al., 2007). Briefly, the grafts were fixed in a customized pressure chamber filled with PBS simulating tissue environment and serving as leakage control. The distal ends of the grafts were sealed, and proximal ends were perfused by a peristaltic pump infusing PBS at a constant rate of 50ml/min until graft burst. The highest pressure measured before failure was defined as the burst strength value.

For determining the elasticity of the grafts, Young's Modulus was measured. The Young's modulus (E) represents a material property and its capability for stretch and deformation. E is defined as the ratio of tensile stress ($\sigma$) to tensile strain ($\epsilon$). Where stress is the amount of force applied per unit area ($\sigma=F/A$) and strain is extension per unit length. E was calculated using the following equations:

$$E = \frac{\sigma}{\epsilon} = \frac{F/A}{dl/l}$$

Tensile and suture strength characteristics of VG and BG-SA-COP were evaluated in a standard tensile test station (Z 0.5 Zwick/Roell, Ulm, Germany). Each vascular graft was mounted on an irregular polymer surface to prevent slippage during the test series (Fixation zone of each graft: 5 mm; Figure 4d + e). After applying the mechanical force (1 mm/min), vascular grafts ruptured near the middle or near both ends. Force measurement was recorded by auditing software testXpert II (Zwick/Roell, Ulm, Germany). For tensile strength testing, a standard synthetic, non-absorbable, monofilament surgical suture (Prolene monofil FS2 6-0, Ethicon, Cincinnati, USA) was placed 3 mm towards the edge of the vascular graft. Both experimental procedures have been described previously (Clements et al., 2007; Konig et al., 2009; Quint et al., 2012; Hoganson et al., 2018).

Results: The systematic comparison of burst strength between VG ($1.82 \times 10^5 \pm 0.29$ Pa), BG-SA ($1.47 \times 10^5 \pm 0.22$ Pa), $BG_{SA-COP2\%}$ ($1.72 \times 10^5 \pm 0.19$ Pa) and $BG_{SA-COP20\%}$ ($1.97 \times 10^5 \pm 0.24$ Pa) revealed significant higher burst strength for $BG_{SA-COP20\%}$ compared to sole SA fabrication (p = 0.00) (Figure 4a + b). Hence, the biomechanical capabilities of both BG-SA-COP for maximal pressure exposure were similar to human VG in this model which is in line with other studies that have described burst strength for vascular grafts ranging between $0.5 - 2.6 \times 10^5$ Pa (Clements et al., 2007; Konig et al., 2009; Quint et al., 2012; Hoganson et al., 2018).

For elasticity analysis of BG-SA-COP, Young's Modulus was determined which was comparable between human VG and both BG-SA-COP (Figure 4c). Calculation of Young's Modulus revealed $771.7 \pm 143.7$ kPa for VG, $710 \pm 130.4$ kPa for $BG_{SA-COP2\%}$ and $761.7 \pm 140.8$ kPa for $BG_{SA-COP20\%}$, respectively (Figure 4c). Consequently Young's Modulus of BG-SA-COP was comparable or appreciably higher than in previous studies examining BG-SA and the elasticity of both BG-SA-COP appeared comparably to human-scale vessels (Clements et al., 2007; Konig et al., 2009; Quint et al., 2012; Zhang et al., 2015; Hoganson et al., 2018) (Figure 4c).

Moreover, suture strength of BG-SA-COP was lower but still comparable to the performance of human VG in the test station (Figure 4d). Suture strength was higher in the VG group ($0.65 \pm 0.10$ N) compared to the $BG_{SA-COP2\%}$ ($0.33 \pm 0.16$ N) and $BG_{SA-COP20\%}$ group ($0.41 \pm 0.25$ N) but only VG and $BG_{SA-COP2\%}$ differed significantly (p = 0.02) (Figure 4d). The reduced suture strength of both BG-SA-COP might be the result of reduced interconnection of fibers in the end sections of the grafts as a consequence of the current fabrication process. Further improvement of this technical issue could be potentially addressed by compression and spinning technologies for fibrin and collagen constructs as reported by various authors. Extension of cross-linking or detaching the end section of the graft could also address this aspect (Helms et al., 2021).

Tensile strength was also similar between VG ($8.96 \pm 1.02$ N) and $BG_{SA-COP2\%}$ ($8.24 \pm 1.74$ N) respectively $BG_{SA-COP20\%}$ ($9.46 \pm 2.60$ N) (Figure 4e). It should also be mentioned that BG-SA broke after few seconds in the test station and therefore no sufficient measurement was possible. Both suture and tensile strength experiments with BG-SA were therefore excluded from the remaining experiments.

Overall, the biomechanical key parameters were comparable between $BG_{SA-COP20\%}$ and VG. The fact that the biomechanical test results for VG are comparable to formerly published studies underline the robustness of the here described experimental set-up (Clements et al., 2007; Konig et al., 2009; Quint et al., 2012; Hoganson et al., 2018).

Example 8: Analysis of dehydration and swelling

The cultured grafts obtained from Example 4 were further analyzed for their dehydration and swelling characteristics. For this purpose, BG-SA and BG-SA-COP were soaked in 4% $CaCl_2$ and culture medium (1:1) for 6 h followed by a dehydration step at room temperature for four days (Zhang et al., 2015). The dehydrated vascular grafts were then soaked in PBS (Sigma Aldrich, U.S.A.) for the evaluation of swelling, Shrinkage Rate by Weight (SRW) and Swelling Ratio (SR). SRW and SR were calculated using the following equations and as described before (Zhang et al., 2015):

$$SRW = \left(1 - \frac{Wd}{W0}\right) x100\% \qquad (1)$$

$$SR = \frac{Wi - Wd}{Wd} x100\% \qquad (2)$$

W0 represents the original weight after fabrication, Wi is the swollen weight at the predetermined time point and Wd is the dehydrated weight.

Results: The test revealed that lumen and total dimension of the grafts were mostly preserved after the dehydration process (Figure 4f). However, $BG_{SA-COP2\%}$ lost more weight as indicated by the Shrinkage Rate by Weight (SRW) (83.78% $\pm$ 12.31%) over time than both BG-SA (81.77 $\pm$ 8.10%) and $BG_{SA-COP20\%}$ (66.48% $\pm$ 6.27%) ($BG_{SA-COP2\%}$ vs $BG_{SA-COP20\%}$, p = 0.03) (Figure 4f). The SRW for the BG-SA group was also comparable to 3-4% alginate tubes described by Zhang and co-workers (Zhang et al., 2015). These findings suggest that SRW decreases with increased COP concentrations. A relatively low SRW also suggests a stable organization on macromolecule level and functional cross-linking which is highly favorable for the here demanded scope of application (Zhang et al., 2015).

The swelling experiments followed the initial dehydration: in accordance, the Swelling Ratio (SR) decreased with increasing proportion of COP in $BG_{SA-COP}$ ($BG_{SA}$: 2399.19% $\pm$ 691.63%; $BG_{SA-COP2\%}$: 1844.42% $\pm$ 261.06%; $BG_{SA-COP20\%}$: 1326.33% $\pm$ 443.03%) respectively (Figure 4g). However, only the comparison between $BG_{SA}$ and $BG_{SA-COP20\%}$ differed significantly (p = 0.01) highlighting the incorporation of collagen in the bio-ink as a significant structural element (Figure 4g).

Example 9: Analysis of coagulation activation and thrombogenicity

To analyse coagulation characteristics of the printed BG-SA-COP, two established models were used: first coagulation activation and solid thrombus formation by whole blood was investigated in the dynamic Chandler Loop model and second platelet activation was analysed by applying Platelet Rich Plasma (PRP) in a platelet-activation-assay (Olsen et al. 2018).

To analyse coagulation activation by the bioprinted grafts, the established Chandler Loop system was used in a slightly modified setting (Olsen et al. 2018). Briefly, in this dynamic in-vitro model, the vascular grafts were integrated into silicon tubes and filled with whole blood from four donors who underwent full coagulation diagnostics prior to the experiments. The vascular grafts then rotated in a constantly warmed water bath at 37°C and maximal clotting formation was assessed by weighing and measuring the thrombus at 30 and 60 min.

Besides full coagulation activation by BG-SA-COP, the distinct activation of platelets by SA and SA-COP was assessed by a platelet-activation-assay applying Platelet Rich Plasma (PRP). 200 $\mu$L of sole SA, SA-COP2% and SA-COP20% were placed on the bottom of a 12 well plate. A solution of 50 % citrated blood and $CaCl_2$ (2mM) was prepared, vortexed for 20 s and centrifuged at 1800 x g for 10 min. The obtained blood plasma was again centrifuged four times at 1800g to receive PRP. Afterwards, 200 $\mu$L of PRP were placed into each well. The control group was composed only by PRP and therefore without surface activation of thrombocytes by SA or SA-COP. The wells were washed with PBS solution at 5, 10 and 15 min to stop clotting and remove soluble clots. Clotting time was defined after first formation of solid clots and number of clots was visually assessed via microscopic examination.

Results: It was found that after 30 min in the Chandler Loop, only the BG-SA showed distinct thrombus formation indicating a higher thrombogenicity than the other experimental groups. After 60 min thrombi occurred in all four experimental groups. No major differences in thrombus weight and size between VG, $BG_{SA-COP2\%}$ and $BG_{SA-COP20\%}$ were observed in these experiments (VG: weight-thrombus = 8.62 $\pm$ 0.88 g; size-thrombus = 29.83 $\pm$ 3.06 mm; $BG_{SA-COP2\%}$: weight-thrombus = 8.23 $\pm$ 0.85 g; size-thrombus = 28.33 $\pm$ 1.63 mm; $BG_{SA-COP20\%}$: weight-thrombus = 7.73 $\pm$ 0.58 g; size-thrombus = 29.50 $\pm$ 3.93 mm) and only comparison of thrombus weight between the BG-SA and $BG_{SA-COP20\%}$ differed significantly (p = 0.03) (Figure 5a-c). Overall, $BG_{SA-COP2\%}$ and $BG_{SA-COP20\%}$ showed a similar performance in in-vitro thrombogenicity compared to human VG. It has been described that exposure of extracellular matrix proteins especially fibrillar collagens I and III are required for thrombus formation and platelet adhesion (Smethurst et al. 2007). However, there was no significant difference in in-vitro thrombogenicity between both BG-SA-COP groups despite the higher percentage of potentially thrombogenic COP in $BG_{SA-COP20\%}$.

The platelet-activation-assay also supported the results from the Chandler Loop model: there was not a significant difference in both SA and the SA-COP groups indicating no enhanced platelet activation by the rising percentage of COP (Figure 5 d). These findings could be due to the highly aligned ultrastructure of the grafts with metabolically active endothelial cells preventing thrombus formation and platelet activation. Although it is commonly accepted that thrombogenicity is positively influenced by the presence of collagen, the thrombus formation was more pronounced in BG-SA than in BG-SA-COP in the Chandler Loop model.

These findings indicate that the properties of collagens, particularly with regard to thrombogenicity, are modified by combination with SA and that the resulting structure has different physical and physiological properties than the original substances alone. The results obtained also confirm the suitability of the grafts produced for putative surgical implantation.

Example 10: Endurance and permeability testing in an in-vitro circulation model

Permeability and initial stability characteristics of the printed vascular grafts were initially assessed by implantation into an established slightly modified in-vitro circulation model consisting of a circulatory pump enabling a consistent circulation of 500 ml NaCl 0.9% fluid tempered at 37°C (Rusch et al., 2019). The vascular grafts were implemented in the circulation model via ligature with vessel loops (Primed, Halberstadt, Germany). A consistent flow rate of 80 ml/min and 100 mmHg simulated the physiological circulation in the coronary artery or small diameter vessels of the lower limb for 5 d (Westerhof et al., 2006). Fluid was collected in a retention basin and flow was consistently assessed via laser doppler flowmetry. After the experiments, the vascular grafts were examined via microscope.

Results: In-vitro testing revealed a low average leakage rate of approximately 0.83 % in the $BG_{SA-COP2\%}$ and 0.57 % in the $BG_{SA-COP20\%}$ group after 5 d in the circulation model (Figure 4a). These results were comparable to an average leakage rate of 0.46 % in the VG group. The higher leakage rate in the $BG_{SA-COP2\%}$ might result from the lower level of interconnected fibres and greater pore size as demonstrated in the SEM imaging (Figure 3a + b). Microscopic inspection did not reveal any structural damage of the grafts after 5 d (data not shown).

Example 11: Surgical implantation in a perfused cadaver model

For initial assessment of surgical suitability of the bioprinted grafts, implantation of the final $BG_{SA-COP20\%}$ in Wistar rats (n = 3) and New Zealand rabbits (n = 3) were performed. Implantation of VG served as control group. Two cardiovascular surgeons performed the end-to-end implantation of the grafts as partial replacement of the aorta. Anastomosis were performed in ongoing suturing and parachute technique with Prolene monofil FS2 6-0 (Ethicon, Cincinnati, USA) (Figure 6a-c). After surgery, permeability of the anastomosis and grafts was tested via on-pump perfusion of the animal aorta with approximately 80 ml/min simulating coronary or crural arterial supply. Evaluation of the completed anastomosis was assessed by applying the slight modified Northwestern Objective Microanastomosis Assessment Tool (NOMAT) focusing only on the sufficiency and quality of anastomosis (Items XII-XIV, range 0-15), subjective (range 0-100) and in-vitro performance grading (pass/fail) made by two independent surgical trained observers via video of the procedures (Table 2) (Aoun et al., 2015).

Results: It was found that surgical implantation in two different perfused cadaver models (New Zealand rabbit n = 3; Wistar rat n = 3) was performed without technical issues even in demanding parachute anastomosis technique (Figure 6a-c). Only one graft failure was observed due to suture tearing. Grading of the performed surgery via the Northwestern Objective Microoanastomosis Assessment Tool (NOMAT) for surgical handling and sufficiency of the vascular anastomosis was comparable to surgery performed with human VG (Table 2). Accordingly, inspection of the anastomosis did not show any sign of high-grade stenosis or leakages after implantation.

Whereas most described tissue-engineered vascular grafts have been acelluar vascular prothesis or preliminary approaches without surgical feasibility, this is the first time that a bioprinted bioartifical vascular graft has been successfully implanted in a surgical model and fulfils all relevant real-world benchmark parameters (Dahl et al., 2011; Zhang et al., 2015; Hong et al., 2019; Cui et al., 2019).

**Table 2:** Results of the implementation of the final vascular grafts in the cadaver model (n = 6). Performance data of the Northwestern Objective Microanastomosis Assessment Tool (NOMAT) grading for the evaluation of anastomoses and graft handling for $BG_{SA-COP20\%}$ in contrast to human Vein Grafts (VG).

|  | SA-COP 20% grafts | human Vein Grafts |
|---|---|---|
| Mean NOMAT (range) | 12.3 (9-15) | 13.5 (11-15) |

(continued)

| | SA-COP 20% grafts | human Vein Grafts |
|---|---|---|
| Mean subjective grade (range) | 70.6 (55-84) | 69 (55-80) |
| In-vitro performance | 5 pass, 1 fail | 6 pass, 0 failed |
| Mean anastomosis time in min (range) | 16.4 (15.4-18.4) | 13.75 (13.4-18.2) |

## LITERATURE

1. Kullo, I.J. and Rooke, T.W., 2016. Peripheral artery disease. New England Journal of Medicine, 374(9), pp.861-871

2. Head, S.J., Milojevic, M., Taggart, D.P. and Puskas, J.D., 2017. Current practice of state-of-the-art surgical coronary revascularization. Circulation, 136(14), pp.1331-1345.

3. Caps, M.T., 1998, December. The epidemiology of vascular trauma. In Seminars in vascular surgery (Vol. 11, No. 4, pp. 227-231).

4. Haney, L.J., Bae, E., Pugh, M.J.V., Copeland, L.A., Wang, C.P., MacCarthy, D.J., Amuan, M.E. and Shireman, P.K., 2020. Patency of arterial repairs from wartime extremity vascular injuries. Trauma surgery & acute care open, 5(1), p.e000616

5. Caliskan, E., De Souza, D. R., Boening, A., Liakopoulos, O. J., Choi, Y. H., Pepper, J., ... & Emmert, M. Y. (2020). Saphenous vein grafts in contemporary coronary artery bypass graft surgery. Nature Reviews Cardiology, 17(3), 155-169.

6. Gallo, M., Trivedi, J. R., Monreal, G., Ganzel, B. L., & Slaughter, M. S. (2020). Risk factors and outcomes in redo coronary artery bypass grafting. Heart, Lung and Circulation, 29(3), 384-389.

7. Xenogiannis, I., Zenati, M., Bhatt, D. L., Rao, S. V., Rodes-Cabau, J., Goldman, S., (...) & Brilakis, E. S. (2021). Saphenous vein graft failure: from pathophysiology to prevention and treatment strategies. Circulation, 144(9), 728-745.

8. Twine, C. P., & McLain, A. D. (2010). Graft type for femoro-popliteal bypass surgery. Cochrane Database of Systematic Reviews, (5).

9. Zhang, Y., Yu, Y., Akkouch, A., Dababneh, A., Dolati, F., & Ozbolat, I. T. (2015). In vitro study of directly bioprinted perfusable vasculature conduits. Biomaterials science, 3(1), 134-143.

10. Hong, S., Kim, J. S., Jung, B., Won, C., & Hwang, C. (2019). Coaxial bioprinting of cell-laden vascular constructs using a gelatin-tyramine bio-ink. Biomaterials science, 7(11), 4578-4587.

11. Sasmal, P., Datta, P., Wu, Y. and Ozbolat, I.T., 2018. 3D bioprinting for modelling vasculature. Microphysiological systems, 2.

12. Cui, H., Zhu, W., Huang, Y., Liu, C., Yu, Z. X., Nowicki, M., ... & Zhang, L. G. (2019). In vitro and in vivo evaluation of 3D bioprinted small-diameter vasculature with smooth muscle and endothelium. Biofabrication, 12(1), 015004.

13. Dahl, S. L., Kypson, A. P., Lawson, J. H., Blum, J. L., Strader, J. T., Li, Y., ... & Niklason, L. E. (2011). Readily available tissue-engineered vascular grafts. Science translational medicine, 3(68), 68ra9-68ra9.

14. Sasmal, P., Datta, P., Wu, Y. and Ozbolat, I.T., 2018. 3D bioprinting for modelling vasculature. Microphysiological systems, 2.

15. Ott, H. C., & Mathisen, D. J. (2011). Bioartificial tissues and organs: are we ready to translate?. Lancet (London, England), 378(9808), 1977-1978.

16. Caliskan, E., De Souza, D. R., Boening, A., Liakopoulos, O. J., Choi, Y. H., Pepper, J., ... & Emmert, M. Y. (2020). Saphenous vein grafts in contemporary coronary artery bypass graft surgery. Nature Reviews Cardiology, 17(3), 155-169.

17. Fan, L., Cao, M., Gao, S., Wang, T., Wu, H., Peng, M., ... & Nie, M. (2013). Preparation and characterization of sodium alginate modified with collagen peptides. Carbohydrate polymers, 93(2), 380-385.

18. Ullah, S., & Chen, X. (2020). Fabrication, applications and challenges of natural biomaterials in tissue engineering. Applied Materials Today, 20, 100656.

19. Hu, T., & Lo, A. C. (2021). Collagen-Alginate Composite Hydrogel: Application in Tissue Engineering and Biomedical Sciences. Polymers, 13(11), 18

20. Matai, I., Kaur, G., Seyedsalehi, A., McClinton, A., & Laurencin, C. T. (2020). Progress in 3D bioprinting technology for tissue/organ regenerative engineering. Biomaterials, 226, 119536.

21. Majesky, M. W. (2018). Vascular development. Arteriosclerosis, thrombosis, and vascular biology, 38(3), e17-e24.

22. Kim, M. J., Park, P. J., Koo, B. H., Lee, S. G., Byun, G. Y., & Lee, S. R. (2020). Association between venous reflux and diameter of great saphenous vein in lower thigh. Journal of Vascular Surgery: Venous and Lymphatic Disorders, 8(1), 100-105.

23. Ketenciler, S., Boyacıoǧlu, K., Akdemir, I., Kömürcü, G., & Polat, A. (2018). Autologous saphenous vein panel graft for vascular reconstruction. *Annals of Vascular Surgery*, 53, 117-122.

24. Konig, G., McAllister, T. N., Dusserre, N., Garrido, S. A., Iyican, C., Marini, A., ... & L'Heureux, N. (2009). Mechanical properties of completely autologous human tissue engineered blood vessels compared to human saphenous vein and mammary artery. Biomaterials, 30(8), 1542-1550.

25. Quint, C., Arief, M., Muto, A., Dardik, A., & Niklason, L. E. (2012). Allogeneic human tissue-engineered blood vessel. Journal of vascular surgery, 55(3), 790-798.

26. Clements, R. H., & Palepu, R. (2007). In vivo comparison of the coagulation capability of SonoSurg and Harmonic Ace on 4 mm and 5 mm arteries. Surgical endoscopy, 21(12), 2203-2206.

27. Hoganson, D. M., Cooper, D. A., Rich, K. N., Piekarski, B. L., Gui, L., Gaut, J. P., ... & Emani, S. M. (2018). Flow Preservation of Umbilical Vein for Autologous Shunt and Cardiovascular Reconstruction. The Annals of thoracic surgery, 105(6), 1809-1818.

28. Olsen, A. L., & Long, M. (2018). Comparison of catheter thrombogenicity in a modified chandler loop model using goat blood. Journal of Biomedical Materials Research Part A, 106(12), 3143-3151.

29. Smethurst, P. A., Onley, D. J., Jarvis, G. E., O'Connor, M. N., Knight, C. G., Herr, A. B., ... & Farndale, R. W. (2007). Structural basis for the platelet-collagen interaction: the smallest motif within collagen that recognizes and activates platelet Glycoprotein VI contains two glycine-proline-hydroxyproline triplets. Journal of Biological Chemistry, 282(2), 1296-1304.

30. Westerhof, N., Boer, C., Lamberts, R. R., & Sipkema, P. (2006). Cross-talk between cardiac muscle and coronary vasculature. Physiological reviews, 86(4), 1263-1308.

31. Rusch, R., Trentmann, J., Hummitzsch, L., Rusch, M., Aludin, S., Haneya, A., ... & Berndt, R. (2019). Feasibility of a circulation model for the assessment of endovascular recanalization procedures and periprocedural thromboembolism in-vitro. Scientific reports, 9(1), 1-6.

32. Helms, F., Haverich, A., Böer, U., & Wilhelmi, M. (2021). Transluminal compression increases mechanical stability, stiffness and endothelialization capacity of fibrin-based bioartificial blood vessels. Journal of the Mechanical Behavior of Biomedical Materials, 124, 104835.

33. Zhang, Y., Yu, Y., Akkouch, A., Dababneh, A., Dolati, F., & Ozbolat, I. T. (2015). In vitro study of directly bioprinted perfusable vasculature conduits. Biomaterials science, 3(1), 134-143.

34. Baudin, B., Bruneel, A., Bosselut, N., & Vaubourdolle, M. (2007). A protocol for isolation and culture of human umbilical vein endothelial cells. Nature protocols, 2(3), 481-485.

35. Mead, L. E., Prater, D., Yoder, M. C., & Ingram, D. A. (2008). Isolation and characterization of endothelial progenitor cells from human blood. Current protocols in stem cell biology, 6(1), 2C-1.

36. Emontzpohl, C., Simons, D., Kraemer, S., Goetzenich, A., Marx, G., Bernhagen, J., & Stoppe, C. (2017). Isolation of endothelial progenitor cells from healthy volunteers and their migratory potential influenced by serum samples after cardiac surgery. Journal of visualized experiments: JoVE, (120).

37. Kong, L., Wang, Y., Wang, H., Pan, Q., Zuo, R., Bai, S., ... & Li, G. (2021). Conditioned media from endothelial progenitor cells cultured in simulated microgravity promote angiogenesis and bone fracture healing. Stem cell research & therapy, 12(1), 1-14.

38. Peters, E. B. (2018). Endothelial progenitor cells for the vascularization of engineered tissues. Tissue Engineering Part B: Reviews, 24(1), 1-24.

39. Jackson, R. J., Dao, M. L., & Lim, D. V. (1995). Modified FALGPA assay for cell-associated collagenolytic activity. Journal of microbiological methods, 21(2), 209-215.

40. Morch, Ý. A., Donati, I., Strand, B. L., & Skjåk-Bræk, G. (2007). Molecular engineering as an approach to design new functional properties of alginate. Biomacromolecules, 8(9), 2809-2814.

41. Xu, Y., Dai, J., Zhu, X., Cao, R., Song, N., Liu, M., ... & Yang, Y. (2021). Biomimetic Trachea Engineering via a Modular Ring Strategy Based on Bone-Marrow Stem Cells and Atelocollagen for use in Extensive Tracheal Reconstruction. Advanced Materials, 2106755.

42. Tamaddon, M., Burrows, M., Ferreira, S. A., Dazzi, F., Apperley, J. F., Bradshaw, A., ... & Gentleman, E. (2017). Monomeric, porous type II collagen scaffolds promote chondrogenic differentiation of human bone marrow mesenchymal stem cells in vitro. Scientific reports, 7(1), 1-10.

43. Su, Y., Toftdal, M. S., Le Friec, A., Dong, M., Han, X., & Chen, M. (2021). 3D Electrospun Synthetic Extracellular Matrix for Tissue Regeneration. Small Science, 2100003.

44. Aoun, S. G., El Ahmadieh, T. Y., El Tecle, N. E., Daou, M. R., Adel, J. G., Park, C. S., & Bendok, B. R. (2015). A pilot study to assess the construct and face validity of the Northwestern Objective Microanastomosis Assessment Tool. Journal of neurosurgery, 123(1), 103-109.

45. Movileanu, I., Harpa, M., Hussam, A.H., Harceaga, L., Chertes, A., Al Hussein, H., Lutter, G., Puehler, T., Preda, T., Sircuta, C. and Cotoi, O., 2021. Pre-clinical Testing of Living Tissue Engineered Heart Valves for Pediatric Patients; Challenges and Opportunities. Frontiers in cardiovascular medicine, 8, p.833.

46. Nakajima, N., & Ikada, Y. (1995). Mechanism of amide formation by carbodiimide for bioconjugation in aqueous media. Bioconjugate chemistry, 6(1), 123-130.

47. Hashimoto, T., Suzuki, Y., Tanihara, M., Kakimaru, K., Suzuki, K. (2004). Development of alginate wound dressings linked with hybrid peptides derived from laminin and elastin. Biomaterials, 25, 1407-1414.

48. Crampton SP, Davis J, Hughes CC (2007). Isolation of human umbilical vein endothelial cells (HUVEC). J Vis Exp;(3):183.

49. Fan, L., Cao, M., Gao, S., Wang, T., Wu, H., Peng, M. & Nie, M. (2013). Preparation and characterization of sodium alginate modified with collagen peptides. Carbohydrate polymers, 93(2), 380-385.

50. Simper, D., Stalboerger, P. G., Panetta, C. J., Wang, S., & Caplice, N. M. (2002). Smooth muscle progenitor cells in human blood. Circulation, 106(10), 1199-1204.

51. Kolster, M., Wilhelmi, M., Schrimpf, C., Hilfiker, A., Ha-verich, A., & Aper, T. (2017). Outgrowing endothelial and smooth muscle cells for tissue engineering approaches. Journal of Tissue Engineering, 8, 2041731417698852.

## Claims

1. Bio-ink composition suitable for being printed into a bioartificial vascular graft, said composition comprising:

   (a) a peptide-grafted alginate; and
   (b) smooth muscle cells, endothelial cells or cells which are capable of developing into endothelial cells.

2. Bio-ink composition according to claim 1, wherein said alginate is sodium alginate.

3. Bio-ink composition according to claim 1 or 2, wherein the overall amount of peptide which is present in the peptide-grafted alginate is between 1% and 25% (w/w), and preferably between 1% and 10% (w/w) based on the overall weight of the alginate.

4. Bio-ink composition according to any of claims 1-3, wherein said endothelial cells are Human Umbilical Vein Endothelial Cells (HUVEC).

5. Bio-ink composition according to any of claims 1-3, wherein said cells which are capable of developing into endothelial cells are Endothelial Progenitor Cells (EPC).

6. Bio-ink composition according to any of claims 1-5, wherein the composition comprises $1\times10^5$ to $1\times10^7$ cells per ml.

7. Bio-ink composition according to any of claims 1-6, wherein the peptide has a size of 3-50 amino acids.

8. Bio-ink composition according to any of claims 1-7, wherein the peptide in the peptide-grafted alginate comprises an amino acid sequence which is derived from or mimics an extracellular matrix protein.

9. Bio-ink composition according to claims 8, wherein the peptide in the peptide-grafted alginate comprises an amino acid sequence which is derived from collagen, laminin, or elastin, and preferably human collagen, laminin, or elastin.

10. Bio-ink composition according to claims 8, wherein

    (a) the peptide in the peptide-grafted alginate comprises amino acid sequences from more than one extracellular matrix protein, and/or
    (b) the alginate is grafted to two or more different peptides.

11. Bio-ink composition according to any of claims 8-10, wherein said peptide comprises the sequence Leu-Gly-Pro-Ala.

12. Bio-ink composition according to any of claims 1-11, wherein the cells are allogenic or autologous in relation to the subject that shall receive the bioartificial vascular graft.

13. Method for manufacturing a bioartificial vascular graft, comprising

    (a) loading the bio-ink composition according to any of claims 1-12 into a 3D printer which is capable of printing viscous hydrogels;
    (b) contacting the bio-ink composition with a crosslinking solution comprising a divalent and/or trivalent cation in the 3D printer to induce crosslinking of the alginate;
    (c) printing the bio-ink to obtain the bioartificial vascular graft.

14. Method according to claim 13, wherein the 3D printer comprises a print head with a first chamber containing the bio-ink composition and a second chamber containing the crosslinking solution, and wherein the fluids of the first and second chamber are mixed upon printing.

15. Method according to claim 13 or 14, wherein the crosslinking solution used in step (b) comprises $Ca^{2+}$ ions.

16. Method according to any of claims 13-15, further comprising a step (d) in which the bioartificial vascular graft obtained from step (c) is transferred into a solution comprising a divalent and/or trivalent cation for further crosslinking.

17. Method according to any of claims 13-16, wherein the bioartificial vascular graft obtained from step (c) or step (d) is transferred in a bioreactor for cell culturing.

18. Use of a bio-ink composition according to any of claims 1-12 for manufacturing a bioartificial vascular graft.

19. Bioartificial vascular graft obtainable by the method of any of claims 13-17.

20. Bioartificial vascular graft according to claim 19 for use in medicine.

21. Bioartificial vascular graft according to claim 19 for use in vascular or cardiovascular therapy, and preferably in vessel repair by or pass surgery.

22. Use of a vascular graft according to claim 19 for in vitro drug screenings or as an in-vitro training model for endovascular or surgical training.

Fig. 1

Fig. 1 - continued

Fig. 2

Fig. 3

Fig. 4

Fig. 5

**Fig. 6**

EPC

HUVEC

Alginate + Collagen peptide
(SA-COP)

Bio-ink formulation

printing

Bypass surgery

Vascular graft

Cell seeding + cultivation for 21 days

**Fig. 7**

Europäisches Patentamt
European Patent Office
Office européen des brevets

# EUROPEAN SEARCH REPORT

**Application Number**

EP 24 15 8239

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/160203 A1 (GATENHOLM PAUL [US]) 30 May 2019 (2019-05-30) * claim 63m67 * | 1-15 | INV. A61L27/20 A61L27/38 A61L27/50 |
| X | GOLUNOVA ANNA ET AL: "Direct and Indirect Biomimetic Peptide Modification of Alginate: Efficiency, Side Reactions, and Cell Response", INTERNATIONAL JOURNAL OF MOLECULAR SCIENCES, vol. 22, no. 11, 27 May 2021 (2021-05-27), page 5731, XP093212962, Basel, CH ISSN: 1422-0067, DOI: 10.3390/ijms22115731 * 'abstract'; figure 1; table 2 * | 1-15 | |
| A | P. SELCAN GUNGOR-OZKERIM ET AL: "Bioinks for 3D bioprinting: an overview", BIOMATERIALS SCIENCE, vol. 6, no. 5, 1 January 2018 (2018-01-01), pages 915-946, XP055708634, GB ISSN: 2047-4830, DOI: 10.1039/C7BM00765E * page 17, paragraph 1 * | 1-15 | |
| X | JIA ZHAOJUN ET AL: "Design, printing, and engineering of regenerative biomaterials for personalized bone healthcare", PROGRESS IN MATERIALS SCIENCE, PERGAMON PRESS, GB, vol. 134, 16 January 2023 (2023-01-16), XP087269996, ISSN: 0079-6425, DOI: 10.1016/J.PMATSCI.2023.101072 [retrieved on 2023-01-16] * table 10 * | 1,4,6-8, 12 | |

TECHNICAL FIELDS SEARCHED (IPC)

A61L

-/--

The present search report has been drawn up for all claims

| Place of search | Date of completion of the search | Examiner |
|---|---|---|
| The Hague | 9 October 2024 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................
& : member of the same patent family, corresponding document

EPO FORM 1503 03.82 (P04C01)

page 1 of 2

**European Patent Office**
Europäisches Patentamt
European Patent Office
Office européen des brevets

## EUROPEAN SEARCH REPORT

**Application Number**

EP 24 15 8239

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/162662 A1 (ALEPH FARMS LTD [IL]) 4 August 2022 (2022-08-04) * claim 32; example 13 * | 1,7,8, 12-15 | |
| A | KARAKAYA EMINE ET AL: "Engineering peptide-modified alginate-based bioinks with cell-adhesive properties for biofabrication", RSC ADVANCES, vol. 14, no. 20, 1 January 2024 (2024-01-01), pages 13769-13786, XP093212959, GB ISSN: 2046-2069, DOI: 10.1039/D3RA08394B * 'abstract' and 'materials and methods' * | 1-15 | |

TECHNICAL FIELDS
SEARCHED       (IPC)

The present search report has been drawn up for all claims

| Place of search | Date of completion of the search | Examiner |
|---|---|---|
| The Hague | 9 October 2024 | Siebum, Bastiaan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

page 2 of 2

**ANNEX TO THE EUROPEAN SEARCH REPORT
ON EUROPEAN PATENT APPLICATION NO.**

EP 24 15 8239

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2024

| Patent document cited in search report | | Publication date | Patent family member(s) | | Publication date |
|---|---|---|---|---|---|
| US 2019160203 | A1 | 30-05-2019 | EP | 3463822 A1 | 10-04-2019 |
| | | | JP | 7177045 B2 | 22-11-2022 |
| | | | JP | 2019518475 A | 04-07-2019 |
| | | | US | 2019160203 A1 | 30-05-2019 |
| | | | WO | 2017210663 A1 | 07-12-2017 |
| WO 2022162662 | A1 | 04-08-2022 | EP | 4284913 A1 | 06-12-2023 |
| | | | IL | 304413 A | 01-09-2023 |
| | | | US | 2024074456 A1 | 07-03-2024 |
| | | | WO | 2022162662 A1 | 04-08-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KULLO, I.J.** ; **ROOKE, T.W.** Peripheral artery disease. *New England Journal of Medicine*, 2016, vol. 374 (9), 861-871
- **HEAD, S.J.** ; **MILOJEVIC, M.** ; **TAGGART, D.P.** ; **PUSKAS, J.D.** Current practice of state-of-the-art surgical coronary revascularization. *Circulation*, 2017, vol. 136 (14), 1331-1345
- **CAPS, M.T.** The epidemiology of vascular trauma. *Seminars in vascular surgery*, December 1998, vol. 11 (4), 227-231
- **HANEY, L.J.** ; **BAE, E.** ; **PUGH, M.J.V.** ; **COPELAND, L.A.** ; **WANG, C.P.** ; **MACCARTHY, D.J.** ; **AMUAN, M.E.** ; **SHIREMAN, P.K.** Patency of arterial repairs from wartime extremity vascular injuries. *Trauma surgery & acute care open*, 2020, vol. 5 (1), e000616
- **CALISKAN, E.** ; **DE SOUZA, D. R.** ; **BOENING, A.** ; **LIAKOPOULOS, O. J.** ; **CHOI, Y. H.** ; **PEPPER, J.** ; **EMMERT, M. Y.** Saphenous vein grafts in contemporary coronary artery bypass graft surgery. *Nature Reviews Cardiology*, 2020, vol. 17 (3), 155-169
- **GALLO, M.** ; **TRIVEDI, J. R.** ; **MONREAL, G.** ; **GANZEL, B. L.** ; **SLAUGHTER, M. S.** Risk factors and outcomes in redo coronary artery bypass grafting. *Heart, Lung and Circulation*, 2020, vol. 29 (3), 384-389
- **XENOGIANNIS, I.** ; **ZENATI, M.** ; **BHATT, D. L.** ; **RAO, S. V.** ; **RODES-CABAU, J.** ; **GOLDMAN, S.** ; **BRILAKIS, E. S.** Saphenous vein graft failure: from pathophysiology to prevention and treatment strategies. *Circulation*, 2021, vol. 144 (9), 728-745
- **TWINE, C. P.** ; **MCLAIN, A. D.** Graft type for femoro-popliteal bypass surgery. *Cochrane Database of Systematic Reviews*, 2010, vol. 5
- **ZHANG, Y.** ; **YU, Y.** ; **AKKOUCH, A.** ; **DABABNEH, A.** ; **DOLATI, F.** ; **OZBOLAT, I. T.** In vitro study of directly bioprinted perfusable vasculature conduits.. *Biomaterials science*, 2015, vol. 3 (1), 134-143
- **HONG, S.** ; **KIM, J. S.** ; **JUNG, B.** ; **WON, C.** ; **HWANG, C.** Coaxial bioprinting of cell-laden vascular constructs using a gelatin-tyramine bio-ink.. *Biomaterials science*, 2019, vol. 7 (11), 4578-4587
- **SASMAL, P.** ; **DATTA, P.** ; **WU, Y.** ; **OZBOLAT, I.T.** 3D bioprinting for modelling vasculature. *Microphysiological systems*, 2018, vol. 2
- **CUI, H.** ; **ZHU, W.** ; **HUANG, Y.** ; **LIU, C.** ; **YU, Z. X.** ; **NOWICKI, M.** ; **ZHANG, L. G.** In vitro and in vivo evaluation of 3D bioprinted small-diameter vasculature with smooth muscle and endothelium. *Biofabrication*, 2019, vol. 12 (1), 015004
- **DAHL, S. L.** ; **KYPSON, A. P.** ; **LAWSON, J. H.** ; **BLUM, J. L.** ; **STRADER, J. T.** ; **LI, Y.** ; **NIKLASON, L. E.** Readily available tissue-engineered vascular grafts.. *Science translational medicine*, 2011, vol. 3 (68), 68ra9-68ra9
- **OTT, H. C.** ; **MATHISEN, D. J.** Bioartificial tissues and organs: are we ready to translate?. *Lancet (London, England)*, 2011, vol. 378 (9808), 1977-1978
- **FAN, L.** ; **CAO, M.** ; **GAO, S.** ; **WANG, T.** ; **WU, H.** ; **PENG, M.** ; **NIE, M.** Preparation and characterization of sodium alginate modified with collagen peptides. *Carbohydrate polymers*, 2013, vol. 93 (2), 380-385
- **ULLAH, S.** ; **CHEN, X.** Fabrication, applications and challenges of natural biomaterials in tissue engineering. *Applied Materials Today*, 2020, vol. 20, 100656
- **HU, T.** ; **LO, A. C.** Collagen-Alginate Composite Hydrogel: Application in Tissue Engineering and Biomedical Sciences. *Polymers*, 2021, vol. 13 (11), 18
- **MATAI, I.** ; **KAUR, G.** ; **SEYEDSALEHI, A.** ; **MCCLINTON, A.** ; **LAURENCIN, C. T.** Progress in 3D bioprinting technology for tissue/organ regenerative engineering. *Biomaterials*, 2020, vol. 226, 119536
- **MAJESKY, M. W.** Vascular development. *Arteriosclerosis, thrombosis, and vascular biology*, 2018, vol. 38 (3), e17-e24
- **KIM, M. J.** ; **PARK, P. J.** ; **KOO, B. H.** ; **LEE, S. G.** ; **BYUN, G. Y.** ; **LEE, S. R.** Association between venous reflux and diameter of great saphenous vein in lower thigh. *Journal of Vascular Surgery: Venous and Lymphatic Disorders*, 2020, vol. 8 (1), 100-105
- **KONIG, G.** ; **MCALLISTER, T. N.** ; **DUSSERRE, N.** ; **GARRIDO, S. A.** ; **LYICAN, C.** ; **MARINI, A.** ; **L'HEUREUX, N.** Mechanical properties of completely autologous human tissue engineered blood vessels compared to human saphenous vein and mammary artery. *Biomaterials*, 2009, vol. 30 (8), 1542-1550

- **QUINT, C.** ; **ARIEF, M.** ; **MUTO, A.** ; **DARDIK, A.** ; **NIKLASON, L. E.** Allogeneic human tissue-engineered blood vessel. *Journal of vascular surgery*, 2012, vol. 55 (3), 790-798
- **CLEMENTS, R. H.** ; **PALEPU, R.** In vivo comparison of the coagulation capability of SonoSurg and Harmonic Ace on 4 mm and 5 mm arteries. *Surgical endoscopy*, 2007, vol. 21 (12), 2203-2206
- **HOGANSON, D. M.** ; **COOPER, D. A.** ; **RICH, K. N.** ; **PIEKARSKI, B. L.** ; **GUI, L.** ; **GAUT, J. P** ; **EMANI, S. M.** Flow Preservation of Umbilical Vein for Autologous Shunt and Cardiovascular Reconstruction. *The Annals of thoracic surgery*, 2018, vol. 105 (6), 1809-1818
- **OLSEN, A. L.** ; **LONG, M.** Comparison of catheter thrombogenicity in a modified chandler loop model using goat blood. *Journal of Biomedical Materials Research Part A*, 2018, vol. 106 (12), 3143-3151
- **SMETHURST, P. A.** ; **ONLEY, D. J.** ; **JARVIS, G. E.** ; **O'CONNOR, M. N.** ; **KNIGHT, C. G.** ; **HERR, A. B.** ; **FARNDALE, R. W.** Structural basis for the platelet-collagen interaction: the smallest motif within collagen that recognizes and activates platelet Glycoprotein VI contains two glycine-proline-hydroxyproline triplets. *Journal of Biological Chemistry*, 2007, vol. 282 (2), 1296-1304
- **WESTERHOF, N.** ; **BOER, C.** ; **LAMBERTS, R. R.** ; **SIPKEMA, P.** Cross-talk between cardiac muscle and coronary vasculature. *Physiological reviews*, 2006, vol. 86 (4), 1263-1308
- **RUSCH, R.** ; **TRENTMANN, J.** ; **HUMMITZSCH, L.** ; **RUSCH, M.** ; **ALUDIN, S.** ; **HANEYA, A.** ; **BERNDT, R.** Feasibility of a circulation model for the assessment of endovascular recanalization procedures and periprocedural thromboembolism in-vitro. *Scientific reports*, 2019, vol. 9 (1), 1-6
- **HELMS, F.** ; **HAVERICH, A.** ; **BÖER, U.** ; **WILHELMI, M.** Transluminal compression increases mechanical stability, stiffness and endothelialization capacity of fibrin-based bioartificial blood vessels. *Journal of the Mechanical Behavior of Biomedical Materials*, 2021, vol. 124, 104835
- **ZHANG, Y.** ; **YU, Y.** ; **AKKOUCH, A.** ; **DABABNEH, A.** ; **DOLATI, F.** ; **OZBOLAT, I. T.** In vitro study of directly bioprinted perfusable vasculature conduits. *Biomaterials science*, 2015, vol. 3 (1), 134-143
- **BAUDIN, B.** ; **BRUNEEL, A.** ; **BOSSELUT, N.** ; **VAUBOURDOLLE, M.** A protocol for isolation and culture of human umbilical vein endothelial cells.. *Nature protocols*, 2007, vol. 2 (3), 481-485
- **MEAD, L. E.** ; **PRATER, D.** ; **YODER, M. C.** ; **INGRAM, D. A.** Isolation and characterization of endothelial progenitor cells from human blood. *Current protocols in stem cell biology*, 2008, vol. 6 (1), 2C-1
- **EMONTZPOHL, C.** ; **SIMONS, D.** ; **KRAEMER, S.** ; **GOETZENICH, A.** ; **MARX, G.** ; **BERNHAGEN, J.** ; **STOPPE, C.** Isolation of endothelial progenitor cells from healthy volunteers and their migratory potential influenced by serum samples after cardiac surgery. *Journal of visualized experiments: JoVE*, 2017, vol. 120
- **KONG, L.** ; **WANG, Y.** ; **WANG, H** ; **PAN, Q.** ; **ZUO, R.** ; **BAI, S.** ; **LI, G.** Conditioned media from endothelial progenitor cells cultured in simulated microgravity promote angiogenesis and bone fracture healing. *Stem cell research & therapy*, 2021, vol. 12 (1), 1-14
- **PETERS, E. B.** Endothelial progenitor cells for the vascularization of engineered tissues. *Tissue Engineering Part B: Reviews*, 2018, vol. 24 (1), 1-24
- **JACKSON, R. J.** ; **DAO, M. L.** ; **LIM, D. V.** Modified FALGPA assay for cell-associated collagenolytic activity. *Journal of microbiological methods,*, 1995, vol. 21 (2), 209-215
- **MORCH, Ý. A.** ; **DONATI, I.** ; **STRAND, B. L.** ; **SKJÅK-BRÆK, G.** Molecular engineering as an approach to design new functional properties of alginate. *Biomacromolecules*, 2007, vol. 8 (9), 2809-2814
- **XU, Y.** ; **DAI, J.** ; **ZHU, X.** ; **CAO, R.** ; **SONG, N.** ; **LIU, M.** ; **YANG, Y.** Biomimetic Trachea Engineering via a Modular Ring Strategy Based on Bone-Marrow Stem Cells and Atelocollagen for use in Extensive Tracheal Reconstruction. *Advanced Materials*, 2021, 2106755
- **TAMADDON, M.** ; **BURROWS, M.** ; **FERREIRA, S. A.** ; **DAZZI, F.** ; **APPERLEY, J. F.** ; **BRADSHAW, A.** ; **GENTLEMAN, E.** Monomeric, porous type II collagen scaffolds promote chondrogenic differentiation of human bone marrow mesenchymal stem cells in vitro. *Scientific reports*, 2017, vol. 7 (1), 1-10
- **SU, Y.** ; **TOFTDAL, M. S.** ; **LE FRIEC, A.** ; **DONG, M.** ; **HAN, X.** ; **CHEN, M.** 3D Electrospun Synthetic Extracellular Matrix for Tissue Regeneration. *Small Science*, 2021, 2100003
- **AOUN, S. G.** ; **El AHMADIEH, T. Y.** ; **El TECLE, N. E.** ; **DAOU, M. R.** ; **ADEL, J. G.** ; **PARK, C. S.** ; **BENDOK, B. R.** A pilot study to assess the construct and face validity of the Northwestern Objective Microanastomosis Assessment Tool. *Journal of neurosurgery*, 2015, vol. 123 (1), 103-109
- **MOVILEANU, I.** ; **HARPA, M.** ; **HUSSAM, A.H.** ; **HARCEAGA, L.** ; **CHERTES, A.** ; **AL HUSSEIN, H.** ; **LUTTER, G.** ; **PUEHLER, T.** ; **PREDA, T.** ; **SIRCUTA, C.** Pre-clinical Testing of Living Tissue Engineered Heart Valves for Pediatric Patients; Challenges and Opportunities. *Frontiers in cardiovascular medicine*, 2021, vol. 8, 833

- **NAKAJIMA, N.** ; **IKADA, Y.** Mechanism of amide formation by carbodiimide for bioconjugation in aqueous media. *Bioconjugate chemistry*, 1995, vol. 6 (1), 123-130
- **HASHIMOTO, T.** ; **SUZUKI, Y.** ; **TANIHARA, M.** ; **KAKIMARU, K.** ; **SUZUKI, K.** Development of alginate wound dressings linked with hybrid peptides derived from laminin and elastin. *Biomaterials*, 2004, vol. 25, 1407-1414
- **CRAMPTON SP** ; **DAVIS J** ; **HUGHES CC**. Isolation of human umbilical vein endothelial cells (HUVEC). *J Vis Exp*, 2007, vol. 3, 183

- **SIMPER, D.** ; **STALBOERGER, P. G.** ; **PANETTA, C. J.** ; **WANG, S.** ; **CAPLICE, N. M.** Smooth muscle progenitor cells in human blood. *Circulation*, 2002, vol. 106 (10), 1199-1204
- **KOLSTER, M.** ; **WILHELMI, M.** ; **SCHRIMPF, C.** ; **HILFIKER, A.** ; **HA-VERICH, A.** ; **APER, T**. Outgrowing endothelial and smooth muscle cells for tissue engineering approaches. *Journal of Tissue Engineering*, 2017, vol. 8